(19) **Europäisches Patentamt European Patent Office Office européen des brevets**

(11) **EP 4 589 004 A1**

(12) **EUROPEAN PATENT APPLICATION**
published in accordance with Art. 153(4) EPC

(43) Date of publication:
**23.07.2025 Bulletin 2025/30**

(21) Application number: **23865547.6**

(22) Date of filing: **13.09.2023**

(51) International Patent Classification (IPC):
**C12N 5/10** (2006.01)        **C07K 5/083** (2006.01)
**C07K 5/087** (2006.01)       **C07K 5/09** (2006.01)
**C07K 5/097** (2006.01)       **C07K 7/06** (2006.01)
**C07K 7/64** (2006.01)        **C12M 1/00** (2006.01)
**C12N 1/00** (2006.01)        **C12N 15/12** (2006.01)
**C12N 15/86** (2006.01)

(52) Cooperative Patent Classification (CPC):
**C07K 5/0804; C07K 5/0812; C07K 5/0815;
C07K 5/0821; C07K 7/06; C07K 7/64;
C07K 14/435; C12M 1/00; C12N 1/00; C12N 5/10;
C12N 15/86**

(86) International application number:
**PCT/JP2023/033295**

(87) International publication number:
**WO 2024/058198 (21.03.2024 Gazette 2024/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **14.09.2022 JP 2022146408
24.05.2023 JP 2023085583**

(71) Applicant: **SEKISUI CHEMICAL CO., LTD.
Osaka-shi
Osaka
530-8565 (JP)**

(72) Inventors:
• **YANAGISAWA, Teruhiko
  Tsukuba-shi, Ibaraki 300-4292 (JP)**
• **HANEDA, Satoshi
  Mishima-gun, Osaka 618-0021 (JP)**
• **IGUCHI, Hiroki
  Mishima-gun, Osaka 618-0021 (JP)**
• **INOUE, Hitoshi
  Tsukuba-shi, Ibaraki 300-4292 (JP)**

(74) Representative: **Ter Meer Steinmeister & Partner
Patentanwälte mbB
Nymphenburger Straße 4
80335 München (DE)**

Remarks:
The complete document including Reference
Table(s) and the Sequence Listing(s) can be
downloaded from the EPO website

(54) **PRODUCTION METHOD FOR INDUCED PLURIPOTENT STEM CELLS**

(57) Provided is a production method for induced pluripotent stem cells in which induced pluripotent stem cells having high undifferentiation and differentiation potency are easily obtained. A production method for induced pluripotent stem cells according to the present invention includes the steps of: introducing an reprogramming factor into somatic cells; and culturing the somatic cells into which the reprogramming factor has been introduced in the presence of a cell scaffold containing a peptide-conjugated polyvinyl acetal resin.

[FIG. 1]

**Description**

**TECHNICAL FIELD**

The present invention relates to a production method for induced pluripotent stem cells.

**BACKGROUND ART**

Induced pluripotent stem cells (iPS cells) have attracted attention in research fields such as academic fields, drug discovery fields, and regenerative medicine fields. The iPS cells can be produced by introducing a reprogramming factor (for example, OCT3/4, SOX2, KLF4, and c-MYC) into somatic cells (for example, Patent Document 1).

As a production method for iPS cells, a method for culturing somatic cells into which a reprogramming factor has been introduced on a laminin-coated plate is widely used (for example, Non-Patent Documents 1 and 2). Laminin plays a role as a cell scaffold.

Meanwhile, Patent Document 2 below discloses a scaffold for cell culture containing a peptide-conjugated polyvinyl alcohol derivative having a polyvinyl alcohol derivative moiety and a peptide moiety. In addition, in Examples of Patent Document 2, it is disclosed that iPS cells were cultured using the scaffold. However, Patent Document 2 does not disclose establishment of iPS cells from the somatic cells.

**Related Art Documents**

**Patent Documents**

Patent Document 1: JP 2019-162054 A
Patent Document 2: WO2020/230885 A1

**Non-Patent Documents**

Non-Patent Document 1: Protocol Human iPS cell culture under feeder-free conditions, CiRA_Ff-iPSC_protocol_JP_v140310 (Kyoto University iPS Cell Research Institute, https://www.cira.kyoto-u.ac.jp/j/research/img/proto col/hipsprotocolFf_140311.pdf, English version URL: https://www.cira.kyoto-u.ac.jp/j/research/img/protocol/ Ff-iPSC-culture_protocol_E_v140311.pdf)
Non-Patent Document 2: (8) iPS Cell Induction Protocol from Human Peripheral Blood Mononuclear Cells and Monocytes Using SRV™ iPSC-4 Vector (TKB_P-008-02) (TOKIWA-Bio inc., http://tokiwa-bio.com/jp/wp-content/ uploads/2022/02/8_%E3%83%92%E3%83%88%E6%9C%AB%E6%A2 %A2%E8%A1%80%E5%8D%98%E6% A0%B8%E7%90%83%E3%83%BB%E5%8D%98%E 7%90%83%E3%81%8B%E3%82%89%E3%81%AEiPS% E7%B4%B0%E8%83%9E%E8% AA%98%E5%B0%8E%E3%83%97%E3%83%AD%E3%83%88%E3%82% B3%E3%83%AB _02.pdf)

**SUMMARY OF THE INVENTION**

**PROBLEMS TO BE SOLVED BY THE INVENTION**

Various attempts have been made to enhance the establishment efficiency of iPS cells having high undifferentiation and differentiation potency. However, it is still difficult to efficiently obtain the iPS cells having high undifferentiation and differentiation potency. When undifferentiation of the iPS cells is low, differentiation may proceed unintentionally during culture of the iPS cells.

An object of the present invention is to provide a production method for induced pluripotent stem cells in which the induced pluripotent stem cells having high undifferentiation and differentiation potency are easily obtained.

**MEANS FOR SOLVING THE PROBLEMS**

According to a broad aspect of the present invention, there is provided a production method for induced pluripotent stem cells, including the steps of: introducing a reprogramming factor into somatic cells; and culturing the somatic cells into which the reprogramming factor is introduced in the presence of a cell scaffold containing a peptide-conjugated polyvinyl acetal resin.

In a specific aspect of the production method for induced pluripotent stem cells according to the present invention, the production method for induced pluripotent stem cells includes: before the step of introducing a reprogramming factor into the somatic cells, a step of culturing somatic cells in the presence of a cell scaffold containing a peptide-conjugated polyvinyl acetal resin.

In a specific aspect of the production method for induced pluripotent stem cells according to the present invention, in the step of introducing the reprogramming factor into the somatic cells, the reprogramming factor is introduced into the somatic cells using a sendaiviral vector.

In a specific aspect of the production method for induced pluripotent stem cells according to the present invention, the reprogramming factor is mRNA.

In a specific aspect of the production method for induced pluripotent stem cells according to the present invention, the mRNA is introduced into the somatic cells by a plasma gene introduction method.

## EFFECT OF THE INVENTION

According to the present invention, it is possible to provide a production method for induced pluripotent stem cells in which induced pluripotent stem cells having high undifferentiation and differentiation potency is easily obtained.

## BRIEF DESCRIPTION OF DRAWINGS

[Fig. 1] Fig. 1 is a diagram illustrating a relationship between the number of passages after somatic cells into which a reprogramming factor has been introduced are cultured and a virus residual rate.

[Fig. 2] Fig. 2 is a diagram illustrating a relationship between the number of culture days after the somatic cells into which the reprogramming factor has been introduced are cultured and the cumulative number of cells.

[Fig. 3] Fig. 3 is a diagram illustrating a relationship between the number of passages after the somatic cell into which the reprogramming factor has been introduced is cultured and the cell viability.

[Fig. 4] Fig. 4 is a micrograph of cells (iPS cells) obtained by culturing the somatic cells into which the reprogramming factor has been introduced. Fluorescence indicates expression of TRA-1-60.

[Fig. 5] Fig. 5 is a diagram illustrating the relationship between the number of passages after the somatic cells into which the reprogramming factor has been introduced are cultured and expression levels of Nanog and Oct3/4.

[Fig. 6] Fig. 6 is a micrograph of cells on Day 39 from a start of differentiation induction of iPS cells. In Examples 1 to 3, the formation of embryoid bodies was observed, and in Comparative Examples 1 to 3, the formation of embryoid bodies was not observed.

[Fig. 7] Fig. 7 is a diagram illustrating the expression levels of endoderm markers (AFP, FOXA2, GATA4, GATA6, SOX17, and SOX7).

[Fig. 8] Fig. 8 is a diagram illustrating the expression levels of mesoderm markers (KDR and T).

[Fig. 9] Fig. 9 is a diagram illustrating the expression levels of ectoderm markers (NES, PAX6, and SOX1).

[Fig. 10] Fig. 10 is a radar chart summarizing differentiation scores to respective germ layers.

[Fig. 11] Fig. 11 is a diagram illustrating a result of an experiment for introducing mRNA (reprogramming factor) into the somatic cells.

[Fig. 12] Fig. 12 is a micrograph in a fluorescence field of cells on Day 14 after mRNA (reprogramming factor) has been introduced into the somatic cells by a plasma gene introduction method.

[Fig. 13] Fig. 13 is a diagram illustrating a relationship between FITC fluorescence intensity and the number of cells.

[Fig. 14] Fig. 14 is a diagram illustrating expression levels (relative quantitative values) of NANOG, OCT3/4, and SOX2.

[Fig. 15] Fig. 15 is a micrograph in a fluorescence field of cells after immunostaining.

[Fig. 16] Fig. 16 is a diagram illustrating the expression levels (relative quantitative values) of endoderm markers (FOXA2, SOX17), mesoderm markers (T), and ectoderm markers (PAX6).

## MODES FOR CARRYING OUT THE INVENTION

Hereinafter, details of the present invention will be described.

A production method for induced pluripotent stem cells according to the present invention includes the steps of: introducing a reprogramming factor into somatic cells; and culturing the somatic cells into which the reprogramming factor has been introduced in the presence of a cell scaffold containing a peptide-conjugated polyvinyl acetal resin.

In the production method for induced pluripotent stem cells according to the present invention, since the above-described configuration is provided, the induced pluripotent stem cells having high undifferentiation and differentiation potency are easily obtained.

In the related art, as a production method for induced pluripotent stem cells, a method for culturing somatic cells into which a reprogramming factor has been introduced on a plate coated with laminin (cell scaffold) has been widely used. The present inventors have found that by culturing the somatic cells into which the reprogramming factor has been introduced in the presence of a cell scaffold containing a peptide-conjugated polyvinyl acetal resin, induced pluripotent stem cells having high undifferentiation and differentiation potency are easily obtained. In particular, in the present invention, it is possible to obtain induced pluripotent stem cells having a high expression level of TRA-1-60, which is an undifferentiated marker, as compared with a known method in which the somatic cells into which the reprogramming factor has been introduced are cultured on the laminin to establish the induced pluripotent stem cells. In addition, in the induced pluripotent stem cells obtained in the present invention, embryoid bodies can be efficiently formed and differentiation potency can be maintained as compared with the induced pluripotent stem cells obtained by culturing the somatic cells into which the reprogramming factor has been introduced on the laminin.

The production method for induced pluripotent stem cells according to the present invention preferably includes a step of culturing somatic cells before the step of introducing the reprogramming factor into the somatic cells.

In the present specification, the "induced pluripotent stem cells" may be referred to as "iPS cells". In the present specification, the "step of culturing somatic cells" provided before the step of introducing a reprogramming factor into the somatic cells may be referred to as "step (1)". In addition, in the present specification, the "step of introducing a reprogramming factor into the somatic cells" may be referred to as "step (2)", and the "step of culturing the somatic cells into which a reprogramming factor has been introduced in the presence of a cell scaffold containing a peptide-conjugated polyvinyl acetal resin" may be referred to as "step (3)".

Therefore, the production method for the iPS cells according to the present invention includes step (2) and step (3). The production method for the iPS cells according to the present invention preferably includes the step (1), step (2), and step (3).

First, the somatic cells and a peptide-conjugated polyvinyl acetal resin that can be used in the present invention will be described.

(Somatic cells)

As the somatic cells, known somatic cells in the related art used for establishing the iPS cells can be used. The somatic cells are preferably animal cells, more preferably mammalian cells, and still more preferably human cells.

Examples of the somatic cells include blood cells, fibroblasts, hepatocytes, pancreatic cells, intestinal epithelial cells, smooth muscle cells, and dental pulp cells. Examples of the origin of the somatic cells include peripheral blood, cord blood, skin tissue, and teeth.

Examples of the blood cells include nucleated cells such as mononuclear cells, neutrophils, eosinophils, basophils, and lymphocytes. The blood cells may be vascular endothelial progenitor cells, blood stem/progenitor cells, T cells, or B cells.

The somatic cells are preferably blood cells or a fibroblast, more preferably blood cells, and still more preferably mononuclear cells.

(Peptide-conjugated polyvinyl acetal resin)

The peptide-conjugated polyvinyl acetal resin has a polyvinyl acetal resin moiety and a peptide moiety. The peptide-conjugated polyvinyl acetal resin is a polyvinyl acetal resin to which a peptide is bonded. In the peptide-conjugated polyvinyl acetal resin, it is preferable that the polyvinyl acetal resin moiety and the peptide moiety are bonded via a linker moiety. Therefore, the peptide-conjugated polyvinyl acetal resin preferably has the polyvinyl acetal resin moiety, the peptide moiety, and the linker moiety.

In the step (3), a cell scaffold containing the peptide-conjugated polyvinyl acetal resin is used. The cell scaffold in the step (3) contains the peptide-conjugated polyvinyl acetal resin. In addition, as described later, the step (1) is preferably a step of culturing somatic cells in the presence of a cell scaffold containing a peptide-conjugated polyvinyl acetal resin. The cell scaffold in the step (1) preferably contains the peptide-conjugated polyvinyl acetal resin.

In the present specification, the peptide-conjugated polyvinyl acetal resin used in the step (1) may be described as a "peptide-conjugated polyvinyl acetal resin (1)". In addition, the polyvinyl acetal resin moiety, the peptide moiety, and the linker moiety of the peptide-conjugated polyvinyl acetal resin (1) may be described as a "polyvinyl acetal resin moiety (1)", a "peptide moiety (1)", and a "linker moiety (1)", respectively.

In the present specification, the peptide-conjugated polyvinyl acetal resin used in the step (3) may be described as a "peptide-conjugated polyvinyl acetal resin (3)". In addition, the polyvinyl acetal resin moiety, the peptide moiety, and the linker moiety included in the peptide-conjugated polyvinyl acetal resin (3) may be described as a "polyvinyl acetal resin moiety (3)", a "peptide moiety (3)", and a "linker moiety (3)", respectively.

In the following description, the common configuration of the peptide-conjugated polyvinyl acetal resin used in the step (1) and the step (3) will be simply described as a "peptide-conjugated polyvinyl acetal resin". In addition, a common description of the polyvinyl acetal resin moiety, the peptide moiety, and the linker moiety of the peptide-conjugated polyvinyl acetal resin used in the step (1) and the step (3) will be simply described as "polyvinyl acetal resin moiety", "peptide moiety", and "linker moiety".

The peptide-conjugated polyvinyl acetal resin (1) and the peptide-conjugated polyvinyl acetal resin (3) may be the same or different. The polyvinyl acetal resin moiety (1) and the polyvinyl acetal resin moiety (3) may be the same or different. The peptide moiety (1) and the peptide moiety (3) may be the same or different. The linker moiety (1) and the linker moiety (3) may be the same or different.

<Polyvinyl acetal resin moiety>

The peptide-conjugated polyvinyl acetal resin has a polyvinyl acetal resin moiety. The polyvinyl acetal resin moiety is a structural portion derived from a polyvinyl acetal resin in the peptide-conjugated polyvinyl acetal resin. The above polyvinyl acetal resin may be used alone, or two or more kinds thereof may be used in combination.

The polyvinyl acetal resin moiety preferably has an acetal group, a hydroxyl group, and an acetyl group in the side chain. However, the polyvinyl acetal resin moiety may not have, for example, an acetyl group. For example, since all of the acetyl groups of the polyvinyl acetal resin moiety are bonded to the linker, the polyvinyl acetal resin moiety may not have an acetyl group.

The polyvinyl acetal resin can be synthesized by acetalizing polyvinyl alcohol with an aldehyde.

The aldehyde used for acetalization of polyvinyl alcohol is not particularly limited. Examples of the aldehyde include an aldehyde having 1 to 10 carbon atoms. The aldehyde may or may not have a chain aliphatic group, a cyclic aliphatic group, or an aromatic group. The aldehyde may be a chain aldehyde or a cyclic aldehyde. The above aldehide may be used alone, or two or more kinds thereof may be used in combination.

From the viewpoint of further enhancing the adhesion between the cell scaffold and the cells and more effectively exhibiting the effect of the present invention, the aldehyde is preferably formaldehyde, acetaldehyde, propionaldehyde, butyraldehyde, or pentanal, and more preferably butyraldehyde. Therefore, the polyvinyl acetal resin is more preferably a polyvinyl butyral resin. The polyvinyl acetal resin moiety (1) is preferably a polyvinyl butyral resin moiety, and the polyvinyl acetal resin moiety (3) is preferably a polyvinyl butyral resin moiety. From the viewpoint of further enhancing the adhesion between the cell scaffold and the cells and more effectively exhibiting the effect of the present invention, the peptide-conjugated polyvinyl acetal resin (1) is preferably a peptide-conjugated polyvinyl butyral resin having a polyvinyl butyral resin moiety and a peptide moiety. From the viewpoint of more effectively exhibiting the effect of the present invention, the peptide-conjugated polyvinyl acetal resin (3) is preferably a peptide-conjugated polyvinyl butyral resin having a polyvinyl butyral resin moiety and a peptide moiety.

In the peptide-conjugated polyvinyl acetal resin (1), the acetalization degree of the polyvinyl acetal resin moiety (1) (the butyralization degree in the case of the polyvinyl butyral resin moiety) is preferably 40 mol% or more, more preferably 50 mol% or more, and preferably 90 mol% or less, more preferably 85 mol% or less. When the acetalization degree is the above lower limit or more, the peptide-conjugated polyvinyl acetal resin (1) is less likely to swell in a culture medium. When the acetalization degree is the above upper limit or less, solubility in a solvent can be improved.

In the peptide-conjugated polyvinyl acetal resin (3), the acetalization degree of the polyvinyl acetal resin moiety (3) (the butyralization degree in a case of the polyvinyl butyral resin moiety) is preferably 40 mol% or more, more preferably 50 mol% or more, and preferably 90 mol% or less, more preferably 85 mol% or less. When the acetalization degree is the above lower limit or more, the peptide-conjugated polyvinyl acetal resin (3) is less likely to swell in the culture medium. When the acetalization degree is the above upper limit or less, solubility in a solvent can be improved.

In the peptide-conjugated polyvinyl acetal resin (1), a content ratio (hydroxyl group amount) of the hydroxyl group of the polyvinyl acetal resin moiety (1) is preferably 15 mol% or more, more preferably 20 mol% or more, and preferably 45 mol% or less, more preferably 30 mol% or less, further preferably 25 mol% or less.

In the peptide-conjugated polyvinyl acetal resin (3), a content ratio (hydroxyl group amount) of the hydroxyl group of the polyvinyl acetal resin moiety (3) is preferably 15 mol% or more, more preferably 20 mol% or more, and preferably 45 mol% or less, more preferably 30 mol% or less, further preferably 25 mol% or less.

In the peptide-conjugated polyvinyl acetal resin (1), the acetylation degree (acetyl group amount) of the polyvinyl acetal resin moiety (1) is preferably 1 mol% or more, more preferably 2 mol% or more, and preferably 5 mol% or less, more preferably 4 mol% or less. When the acetylation degree is the above lower limit or more and the above upper limit or less, the reaction efficiency between the polyvinyl acetal resin and the linker can be enhanced.

In the peptide-conjugated polyvinyl acetal resin (3), the acetylation degree (acetyl group amount) of the polyvinyl acetal resin moiety (3) is preferably 1 mol% or more, more preferably 2 mol% or more, and preferably 5 mol% or less, more preferably 4 mol% or less. When the acetylation degree is the above lower limit or more and the above upper limit or less, the reaction efficiency between the polyvinyl acetal resin and the linker can be enhanced.

The acetalization degree, the acetylation degree and the hydroxyl group amount of the polyvinyl acetal resin moiety can be measured by $^1$H-NMR (nuclear magnetic resonance spectrum).

<Peptide moiety>

The peptide-conjugated polyvinyl acetal resin has a peptide moiety. The peptide moiety is a structural moiety derived from a peptide in the peptide-conjugated polyvinyl acetal resin. The peptide moiety has an amino acid sequence. The peptide constituting the peptide moiety may be an oligopeptide or a polypeptide. The above peptide may be used alone, or two or more kinds thereof may be used in combination.

The number of amino acid residues in the peptide moiety (1) is preferably 3 or more, more preferably 4 or more, further preferably 5 or more, and preferably 10 or less, more preferably 8 or less, further preferably 6 or less. When the number of amino acid residues is the above lower limit or more and the above upper limit or less, the adhesion to cells after seeding can be further enhanced, and a proliferation rate of cells can be further enhanced. However, the number of amino acid residues in the peptide moiety (1) may be more than 10 or more than 15.

The number of amino acid residues in the peptide moiety (3) is preferably 3 or more, more preferably 4 or more, further preferably 5 or more, and preferably 10 or less, more preferably 8 or less, further preferably 6 or less. When the number of amino acid residues is the above lower limit or more and the above upper limit or less, the adhesion to cells after seeding can be further enhanced, and a proliferation rate of cells can be further enhanced. However, the number of amino acid residues in the peptide moiety (3) may be more than 10 or more than 15.

The peptide moiety (1) preferably has a cell adhesive amino acid sequence. The peptide moiety (3) preferably has a cell adhesive amino acid sequence. The cell adhesive amino acid sequence refers to an amino acid sequence whose cell adhesion activity has been confirmed by a phage display method, a sepharose bead method, or a plate coating method. As the phage display method, for example, the method described in "The Journal of Cell Biology, Volume 130, Number 5, September 1995 1189-1196" can be used. As the sepharose bead method, for example, the method described in "Protein nucleic acid enzyme Vol. 45 No. 15 (2000) 2477" can be used. As the plate coating method, for example, the method described in "Protein nucleic acid enzyme Vol. 45 No. 15 (2000) 2477" can be used.

Examples of the cell adhesive amino acid sequence include an RGD sequence (Arg-Gly-Asp), a YIGSR sequence (Tyr-Ile-Gly-Ser-Arg), a PDSGR sequence (Pro-Asp-Ser-Gly-Arg), a HAV sequence (His-Ala-Val), an ADT sequence (Ala-Asp-Thr), a QAV sequence (Gln-Ala-Val), an LDV sequence (Leu-Asp-Val), an IDS sequence (Ile-Asp-Ser), an REDV sequence (Arg-Glu-Asp-Val), an IDAPS sequence (Ile-Asp-Ala-Pro-Ser), a KQAGDV sequence (Lys-Gln-Ala-Gly-Asp-Val), and a TDE sequence (Thr-Asp-Glu). Examples of the cell adhesive amino acid sequence include the sequences described in "Pathophysiology, Vol. 9 No. 7, p. 527 to 535, 1990" and "Journal of Osaka Women's and Children's Hospital, Vol. 8, No. 1, p. 58 to 66, 1992". The peptide moiety may have only one type of the cell adhesive amino acid sequence or two or more kinds thereof.

The cell adhesive amino acid sequence preferably has at least one of the above-described cell adhesive amino acid sequences, more preferably has at least an RGD sequence, a YIGSR sequence, or a PDSGR sequence, further preferably has an RGD sequence, and particularly preferably has at least an RGD sequence represented by the following Formula (1). In this case, the adhesion to the cells after seeding can be further enhanced, and the proliferation rate of cells can be further enhanced.

$$\text{Arg-Gly-Asp-X} \dots \qquad \text{Formula (1)}$$

In the above Formula (1), X represents Gly, Ala, Val, Ser, Thr, Phe, Met, Pro, or Asn.

The peptide moiety (1) may be linear or may have a cyclic peptide skeleton. The peptide moiety (3) may be linear or may have a cyclic peptide skeleton. The cyclic peptide skeleton is a cyclic skeleton constituted by a plurality of amino acids. From the viewpoint of more effectively exhibiting the effects of the present invention, the cyclic peptide skeleton is preferably constituted by 4 or more amino acids, more preferably constituted by 5 or more amino acids, and preferably constituted by 10 or less amino acids.

In the peptide-conjugated polyvinyl acetal resin (1), the content ratio of the peptide moiety (1) is preferably 0.05 mol% or more, more preferably 0.1 mol% or more, further preferably 0.15 mol% or more, particularly preferably 0.2 mol% or more, preferably 25 mol% or less, more preferably 20 mol% or less, further preferably 15 mol% or less, and particularly preferably 10 mol% or less. When the content ratio of the peptide moiety (1) is the above lower limit or more, the adhesion to cells after seeding can be further enhanced, and the proliferation rate of cells can be further enhanced. When the content ratio of the peptide moiety (1) is the above upper limit or less, the production cost can be suppressed.

In the peptide-conjugated polyvinyl acetal resin (3), the content ratio of the peptide moiety (3) is preferably 0.05 mol% or more, more preferably 0.1 mol% or more, further preferably 0.15 mol% or more, particularly preferably 0.2 mol% or more, preferably 25 mol% or less, more preferably 20 mol% or less, further preferably 15 mol% or less, and particularly preferably 10 mol% or less. When the content ratio of the peptide moiety (3) is the above lower limit or more, the adhesion to cells after seeding can be further enhanced, and the proliferation rate of cells can be further enhanced. When the content ratio of the peptide moiety (3) is the above upper limit or less, the production cost can be suppressed.

The content ratio (mol%) of the peptide moiety is the substance amount of the peptide moiety with respect to the sum of the substance amounts of the respective structural units constituting the peptide-conjugated polyvinyl acetal resin.

The content ratio of the peptide moiety can be measured by, for example, NMR (nuclear magnetic resonance).

<Linker moiety>

The peptide-conjugated polyvinyl acetal resin preferably has a linker moiety. The linker moiety is a structural moiety derived from a linker in the peptide-conjugated polyvinyl acetal resin. The linker moiety is usually located between the polyvinyl acetal resin moiety and the peptide moiety. The polyvinyl acetal resin moiety and the peptide moiety are bonded via the linker moiety. The linker moiety is formed by a linker (crosslinking agent) . The above linker may be used alone, or two or more kinds thereof may be used in combination.

The linker is preferably a compound having a functional group capable of bonding to the peptide, and more preferably a compound having a functional group capable of condensing with a carboxyl group or an amino group of the peptide.

Examples of the functional group capable of condensing with a carboxyl group or an amino group of the peptide include a carboxyl group, a thiol group, an amino group, a hydroxyl group, and a cyano group.

From the viewpoint of favorably reacting with the peptide, the linker is preferably a compound having a carboxyl group or an amino group, and more preferably a compound having a carboxyl group.

Examples of the linker having a carboxyl group include (meth)acrylic acid and a carboxyl group-containing acrylamide. By using carboxylic acid (carboxylic acid monomer) having a polymerizable unsaturated group as the linker having a carboxyl group, the carboxylic acid monomer can be polymerized by graft polymerization at the time of introduction of the linker, so that the number of carboxyl groups that can react with the peptide can be increased.

From the viewpoint of favorably bonding the polyvinyl acetal resin and the peptide, the linker is preferably (meth) acrylic acid, and more preferably acrylic acid.

<Other details of peptide-conjugated polyvinyl acetal resin>

The number average molecular weight of the peptide-conjugated polyvinyl acetal resin (1) is preferably 10,000 or more, more preferably 50,000 or more, further preferably 100,000 or more, and preferably 5 million or less, more preferably 2.5 million or less, further preferably 1 million or less. When the number average molecular weight is the lower limit or more, the cell scaffold is less likely to be eluted into the liquid culture medium during cell culture, and the cell scaffold is less likely to be detached from the container or the like during the cell culture. When the number average molecular weight is the above upper limit or less, the solubility in an alcohol solvent can be enhanced.

The number average molecular weight of the peptide-conjugated polyvinyl acetal resin (3) is preferably 10,000 or more, more preferably 50,000 or more, further preferably 100,000 or more, and preferably 5 million or less, more preferably 2.5 million or less, further preferably 1 million or less. When the number average molecular weight is the lower limit or more, the cell scaffold is less likely to be eluted into the liquid culture medium during cell culture, and the cell scaffold is less likely to be detached from the container or the like during the cell culture. When the number average molecular weight is the above upper limit or less, the solubility in an alcohol solvent can be enhanced.

The number average molecular weight of the peptide-conjugated polyvinyl acetal resin can be measured, for example, by the following method. The peptide-conjugated polyvinyl acetal resin is dissolved in tetrahydrofuran (THF) to prepare a 0.2 wt% solution of the peptide-conjugated polyvinyl acetal resin. Next, evaluation is performed under the following measurement conditions using a gel permeation chromatography (GPC) measuring device (APC system, manufactured by Waters Corporation).

Column: HSPgel HR MB-M 6.0 × 150 mm
Flow rate: 0.5 mL/min
Column temperature: 40°C
Injection amount: 10 μL
Detector: RI, PDA
Standard sample: Polystyrene

Examples of the method for obtaining the peptide-conjugated polyvinyl acetal resin include the following methods.

A polyvinyl acetal resin and a linker are reacted to obtain a reactant in which the polyvinyl acetal resin and the linker are bonded. The obtained reactant is reacted with a peptide to obtain a peptide-conjugated polyvinyl acetal resin.

The culture vessel including the cell scaffold containing the peptide-conjugated polyvinyl acetal resin can be produced, for example, by the following method (A) or (B). (A) A liquid containing the peptide-conjugated polyvinyl acetal resin and a solvent is applied onto a surface of a vessel (culture vessel body), and then the solvent is volatilized. (B) A layer containing a reactant in which a polyvinyl acetal resin and a linker are bonded is disposed on a surface of a vessel (culture vessel body). A liquid containing a peptide is added onto the surface of the layer containing the reactant to react the reactant with the peptide.

Hereinafter, the details of the production method for the iPS cells according to the present invention will be further described.

(Step (1))

The production method for iPS cells preferably includes the step of culturing somatic cells (step (1)). The step (1) is preferably a step of pre-culturing somatic cells. The step (1) may be a step of culturing the somatic cells in the absence of the cell scaffold, or may be a step of culturing the somatic cells in the presence of the cell scaffold. The step (1) may be a step of subjecting somatic cells to suspension culture. From the viewpoint of effectively exhibiting the effect of the present invention, the step (1) is preferably a step of culturing the somatic cells in the presence of the cell scaffold. The cell scaffold is disposed, for example, on the surface of a culture substrate body. The step (1) is, for example, a step of culturing somatic cells using a culture substrate including the culture substrate body and the cell scaffold.

The shape and size of the culture substrate body are not particularly limited. Examples of the culture substrate body include a container, a fiber, a nonwoven fabric, a hollow fiber, a particle, a film, and a porous membrane.

The culture substrate used in the step (1) is preferably a culture vessel. In this case, the cell scaffold is preferably disposed on the bottom surface of the culture vessel body. The culture vessel may be a culture dish or a plate having a plurality of wells.

Examples of the cell scaffold used in the step (1) include a peptide-conjugated polyvinyl acetal resin, laminin, vitronectin, and collagen. Examples of commercially available products of the cell scaffold used in the step (1) include "iMatrix-511" manufactured by Nippi. Inc., "Matrigel" manufactured by Corning Incorporated, and the like.

From the viewpoint of more effectively exhibiting the effect of the present invention, the step (1) is preferably a step of culturing somatic cells in the presence of a cell scaffold containing a peptide-conjugated polyvinyl acetal resin. When the somatic cells are cultured in the presence of the cell scaffold containing the peptide-conjugated polyvinyl acetal resin in the step (1), there are the somatic cells adhering to the cell scaffold and the somatic cells floating without adhering to the cell scaffold. The reason why the effect of the present invention is more effectively exerted by using the cell scaffold is not clear, but it is considered that a useful cytokine may be secreted from the somatic cells adhering to the cell scaffold containing the peptide-conjugated polyvinyl acetal resin. However, the above is estimation, and the reason why the effect of the present invention is more effectively exerted by using the cell scaffold containing the peptide-conjugated polyvinyl acetal resin in the step (1) is not limited thereto.

In the cell scaffold used in the step (1), the peptide-conjugated polyvinyl acetal resin is preferably present at least on a surface that adheres to the somatic cells (adhesion surface with somatic cells).

As a culture medium for culturing the somatic cells in the step (1), a known culture medium in the related art can be used. The culture medium is preferably a liquid culture medium. As the culture medium, a commercially available culture medium may be used, or a self-prepared culture medium may be used. As the culture medium, for example, the culture medium disclosed in Non-Patent Document 1 described above can be used.

Examples of commercially available products of the culture medium include "Stem Fit" manufactured by Ajinomoto Corporation, "StemSpan-ACF", "TeSR E8" and "mTeSR1" manufactured by STEMCELL Technologies Inc., "X-VIVO 10" manufactured by Lonza Corporation, and "StemFlex" and "Essential 8" manufactured by Thermo Fisher Scientific. As the culture medium, a culture medium obtained by adding various cytokines to the commercially available product may be used.

When mononuclear cells are used as the somatic cells, a culture medium containing IL-6 at 45 to 55 ng/mL, SCF at 45 to 55 ng/mL, TPO at 9 to 11 ng/mL, Flt-3L at 18 to 22 ng/mL, IL-3 at 18 to 22 ng/mL, and G-CSF at 9 to 11 ng/mL is suitably used as the final concentration of cytokines. The culture medium having the cytokine concentration can be obtained, for example, by adding these cytokines to the commercially available product so as to have the final concentration.

The culture temperature in the step (1) is preferably 35°C or higher and preferably 38°C or lower. The $CO_2$ concentration during culture in the step (1) is preferably 4% or more and preferably 6% or less. For example, the culture conditions in the step (1) are conditions of 37°C and a $CO_2$ concentration of 5%. The culture time in the step (1) can be, for example, 4 days to 6 days (preferably 5 days). At the time of culture in the step (1), the culture medium may or may not be replaced. When the culture medium is replaced, for example, it can be replaced with a fresh culture medium at a frequency of once a day.

(Step (2))

The production method for iPS cells includes a step (step (2)) of introducing a reprogramming factor into the somatic cells. In the step (2), it is preferable to introduce the reprogramming factor into the somatic cells obtained in the step (1).

From the viewpoint of more effectively exhibiting the effect of the present invention, the step (2) preferably includes a step of collecting the somatic cells after the step (1) and a step of introducing the reprogramming factor into the collected somatic cells.

In the step of collecting the somatic cells, the somatic cells adhered to the cell scaffold after the step (1) may be collected, the somatic cells not adhered to the cell scaffold after the step (1) may be collected, or the somatic cells adhered to the cell scaffold after the step (1) and the somatic cells not adhered to the cell scaffold after the step (1) may be collected. In the step of collecting the somatic cells, it is preferable that the solution (liquid containing somatic cells) in the culture vessel is pipetted and collected in a tube. Thereafter, the liquid obtained by adding DPBS (-) into the culture vessel may or may not be recovered in the tube from which the above solution was recovered.

As the reprogramming factor (nuclear reprogramming factor), the known reprogramming factor in the related art can be used.

Examples of the gene included in the reprogramming factor include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, and Glis1. The above reprogramming factor may be used alone, or two or more kinds thereof may be used in combination.

The reprogramming factor preferably includes an Oct family gene, a Sox family gene, a Klf family gene, a Myc family gene, a Lin28 family gene, or a Nanog family gene.

The reprogramming factor more preferably includes an Oct family gene, a Klf family gene, and a Sox family gene, further preferably includes Oct3/4, Sox2, and Klf4, still more preferably includes Oct3/4, Sox2, Klf4, and c-Myc, and even more preferably includes Oct3/4, Sox2, Klf4, c-Myc, Lin28, and Nanog.

As a method for introducing the reprogramming factor into the somatic cells, a known introduction method in the related art can be used.

Examples of the method for introducing the reprogramming factor into the somatic cells include a method for using a vector and a method for using mRNA. Examples of the vector include viral vectors such as a sendaiviral vector, a retroviral vector, a lentiviral vector, an adenoviral vector, and an adeno-associated viral vector, and non-viral vectors such as an episomal vector and a plasmid vector.

Methods using vectors:

In the method for introducing the reprogramming factor into the somatic cells, it is preferable to use a vector, and it is more preferable to use a sendaiviral vector. That is, in the step (2), it is more preferable to introduce the reprogramming factor into the somatic cells using the sendaiviral vector. In the case of using the sendaiviral vector, safety can be enhanced.

Examples of commercially available products of the sendaiviral vector include "CytoTune-iPS" series manufactured by ID Pharma, "SRV iPSC Vector" series manufactured by Tokiwa Bio Inc., and the like.

In the step (2), it is preferable to infect a viral vector (particularly a sendaiviral vector) with an MOI (Multiplicity of Infection) of 1 or more and 6 or less, and it is more preferable to infect the viral vector with an MOI of 3 or more and 4 or less. The infection time is preferably 15 minutes or more and 4 hours or less, and more preferably 30 minutes or more and 4 hours or less.

In the step (2) using the vector, the process may proceed to the step (3) without culturing the cells into which the reprogramming factor has been introduced after introducing the reprogramming factor into the somatic cells, or the process may proceed to the step (3) through the step of culturing the somatic cells into which the reprogramming factor has been introduced after introducing the reprogramming factor into the somatic cells. The step of culturing the somatic cells into which the reprogramming factor is introduced in the step (2) is culture performed in the absence of a cell scaffold containing a peptide-conjugated polyvinyl acetal resin. The culture time in the step of culturing the somatic cells into which the reprogramming factor is introduced in the step (2) is preferably 7 days or more and preferably 30 days or less.

Method using mRNA:

In the method for introducing the reprogramming factor into the somatic cells, it is also preferable to use mRNA.

The reprogramming factor is also preferably mRNA. In this case, since mRNA (reprogramming factor) can be introduced into the somatic cells without using a virus, safety can be enhanced.

Examples of the method for introducing the mRNA (reprogramming factor) into the somatic cells include a plasma gene introduction method, an electroporation method, and a lipofection method.

The method for introducing the mRNA (reprogramming factor) into the somatic cells is preferably a plasma gene introduction method. That is, the mRNA (reprogramming factor) is preferably introduced into the somatic cells by a plasma gene introduction method. In this case, the mRNA (reprogramming factor) can be introduced into the somatic cells while damage to the genome of the somatic cell is suppressed. As a condition of plasma irradiation in the plasma gene introduction method, known conditions in the related art can be employed.

The introduction of the mRNA (reprogramming factor) into the somatic cells is preferably performed in the presence of a cell scaffold containing a peptide-conjugated polyvinyl acetal resin. It is preferable to introduce the mRNA (reprogramming factor) into the somatic cells adhering to the cell scaffold containing the peptide-conjugated polyvinyl acetal resin. In this case, although the reason is not clear, the introduction efficiency of the mRNA into the somatic cells can be further enhanced as compared with the case of performing in the presence of a cell scaffold containing a protein.

In the step (2) using the mRNA, the step (1) and the step (2) may be performed using the same culture substrate, the step (2) and the step (3) may be performed using the same culture substrate, or the step (1), the step (2), and the step (3) may be performed using the same culture substrate.

(Step (3))

The production method for iPS cells includes a step of culturing somatic cells into which a reprogramming factor has been introduced in the presence of a cell scaffold containing a peptide-conjugated polyvinyl acetal resin (step (3)). In the step (3), the somatic cells obtained in the step (2) are cultured in the presence of the cell scaffold. The cell scaffold is disposed, for example, on the surface of a culture substrate body. The step (3) is, for example, a step of culturing the somatic cells using the culture substrate including the culture substrate body and the cell scaffold.

The shape and size of the culture substrate body are not particularly limited. Examples of the culture substrate body include a container, a fiber, a nonwoven fabric, a hollow fiber, a particle, a film, and a porous membrane.

The culture substrate used in the step (3) is preferably a culture vessel. In this case, the cell scaffold is preferably disposed on the bottom surface of the culture vessel body. The culture vessel may be a culture dish or a plate having a plurality of wells.

The cell scaffold used in the step (3) contains a peptide-conjugated polyvinyl acetal resin. In the cell scaffold used in the step (3), the peptide-conjugated polyvinyl acetal resin is preferably present at least on a surface that adheres to somatic cells (adhesion surface with somatic cells).

In the step (3), the somatic cells into which the reprogramming factor has been introduced are preferably cultured on the surface of the cell scaffold. In the step (3), adhesion culture is preferably performed.

As a culture medium for culturing the somatic cells in the step (3), a known culture medium in the related art can be used. The culture medium is preferably a liquid culture medium. As the culture medium, a commercially available culture medium may be used, or a self -prepared culture medium may be used. As the culture medium, for example, the culture medium disclosed in Non-Patent Document 1 described above can be used.

Examples of commercially available products of the culture medium include "Stem Fit" manufactured by Ajinomoto Corporation, "StemSpan-ACF", "TeSR-E8" and "mTeSR1" manufactured by STEMCELL Technologies Inc., "X-VIVO 10" manufactured by Lonza Corporation, and "StemFlex" and "Essential 8" manufactured by Thermo Fisher Scientific. As the culture medium, a culture medium obtained by adding various cytokines to the commercially available product may be used.

The culture temperature in the step (3) is preferably 36°C or higher and preferably 38°C or lower. The $CO_2$ concentration during culture in the step (3) is preferably 4% or more and preferably 6% or less. For example, the culture conditions in the step (3) are conditions of 37°C and a $CO_2$ concentration of 5%. In addition, at the time of culture in the step (3), the culture medium may or may not be replaced. When the culture medium is replaced, for example, the culture medium can be replaced with a fresh culture medium at a frequency of once every two days.

The iPS cells are induced in, for example, about 14 days from the start of culture in the step (3), and a cell population containing the iPS cells can be obtained.

Whether or not the somatic cells have been induced into the iPS cells can be confirmed, for example, by observing the morphology of the cells or analyzing whether or not undifferentiated markers are expressed in the cells by a flow cytometer. As the undifferentiated marker, TRA-1-60, SSEA4, and the like are known. In particular, since TRA-1-60 is an antigen specific to the iPS cells or ES cells and is not detected in the somatic cells, it is a suitable marker for confirming whether it is induced in the iPS cells or not (for example, WO2018/155595 A1, JP 2019-162054 A, Elayne M Chan1 et al. (Live cell imaging distinguishes bona fide human iPS cells from partially reprogrammed cells, nature biotechnology volume 27 number 11 november 2009)).

Hereinafter, the present invention will be specifically described with reference to examples and comparative examples. The present invention is not limited only to the following examples.

The following were prepared as culture vessels.

Culture vessel (S) (a plate provided with a cell scaffold containing a peptide-conjugated polyvinyl acetal resin, produced by the following production method (S))

Culture vessel (L) (a plate coated with "Laminin 511E8 fragment (iMatrix-511)" manufactured by Nippi. Inc., "mircroplate with a lid, Flat bottom, TC treated polystylene" manufactured by IWAKI)

Culture vessel (F) (a suspension culture plate, "suspension culture plate" manufactured by Corning Incorporated)

[Preparation method for culture vessel (S)]

Preparation of coating liquid containing peptide-conjugated polyvinyl acetal resin:

As a polyvinyl acetal resin, a polyvinyl butyral resin having an acetalization degree (butyralization degree) of 65 mol%, a hydroxyl group amount of 32 mol%, and an acetyl group amount of 3 mol% was prepared. 30 parts by weight of acrylic acid and 70 parts by weight of a polyvinyl acetal resin were dissolved in 255 parts by weight of tetrahydrofuran to obtain a polymer mixed solution. 0.015 parts by weight of Perbutyl O (manufactured by NOF CORPORATION) was dissolved in the obtained polymer mixture solution, and the mixture was reacted at 90°C for 6 hours. Then, the solution after the reaction was mixed with 30,000 parts by weight of water. The obtained precipitate was vacuum-dried at 80°C for 3 hours to produce a polyvinyl acetal resin (polyvinyl butyral resin having a linker) having a linker (acrylic acid). Hereinafter, the "polyvinyl acetal resin having a linker" may be referred to as the "polyvinyl acetal resin (X)".

Dimethylformamide (DMF) was prepared as a first solvent. Dimethylformamide (DMF) was prepared as a second solvent. As a peptide, a cyclic peptide having an amino acid sequence of Arg-Gly-Asp-Phe-Lys (the number of amino acid residues is 5, Arg and Lys are bonded to form a cyclic skeleton, and Phe is a D-form.) was prepared. As a condensing agent, 1-ethyl-3-(3-dimethylaminopropyl) carbodiimide hydrochloride was prepared. A first solution was prepared by mixing 1,000 parts by weight of the first solvent with 50 parts by weight of the polyvinyl acetal resin (X) and 2 parts by weight of the peptide. In addition, 1 part by weight of a condensing agent was mixed with 1,000 parts by weight of the second solvent to prepare a second solution. The first solution and the second solution were mixed to prepare a solution containing the polyvinyl acetal resin (X), the peptide, and the condensing agent.

The obtained solution was reacted at 40°C for 2 hours to dehydrate and condense the carboxyl group in the structural unit derived from acrylic acid of the polyvinyl acetal resin (X) and the amino group of Lys of the peptide to obtain a solution containing a peptide-conjugated polyvinyl acetal resin.

The obtained solution containing the peptide-conjugated polyvinyl acetal resin was diluted 100 times with DMF, and the diluted solution was dropped onto a column packed with an ion exchange resin (manufactured by Organo Corporation) at a rate of 0.3 mL/min and washed. The solution after washing was vacuum-dried at 60°C for 3 hours to obtain a dried solid, and the dried solid was dissolved in butanol (alcohol solvent). Thereafter, 5 parts by weight of acetic acid (pH adjusting agent) was added to 100 parts by weight of butanol (alcohol solvent) to obtain a coating liquid containing a peptide-conjugated polyvinyl acetal resin and butanol. The content of the peptide-conjugated polyvinyl acetal resin in the coating liquid was 0.1 wt%.

Preparation of culture vessel (S):

100 μL of the obtained coating liquid was applied to each well of a 6-well plate (bottom area of each well: 9.5 cm$^2$) by a cast coating method, and then the alcohol solvent was removed by vacuum drying at 60°C for 3 hours. In this way, the culture vessel (S) in which the cell scaffold containing the peptide-conjugated polyvinyl acetal resin (resin film which is a dried solid layer of the coating liquid) was disposed on the bottom surface of each well was obtained. In the peptide-conjugated polyvinyl acetal resin, the content ratio of the peptide moiety was 1 mol%. The number average molecular weight of the peptide-conjugated polyvinyl acetal resin was 50,000.

The following culture media were prepared.

Culture medium (1) (prepared by the following preparation method (1), corresponding to the mononuclear cell culture medium for non-Tcell disclosed in Non-Patent Document 1)
Culture Medium (2) (prepared by the following preparation method (1), corresponding to the mononuclear cell culture medium for non-Tcell disclosed in Non-Patent Document 1, and identical to the culture medium (1))
Culture Medium (3) (prepared by the following preparation method (3), corresponding to the maintenance culture medium StemFit disclosed in Non-Patent Document 1)

[Preparation method for culture medium (1)]

"Stem Fit AK02N solution A" (400 mL) manufactured by Ajinomoto Corporation and "Stem Fit AK02N solution B" (100 mL) manufactured by Ajinomoto Corporation were mixed. To the obtained mixed solution, IL-6, SCF, TPO, Flt-3L, IL-3, and G-CSF were added at final concentrations of 50 ng/mL, 50 ng/mL, 10 ng/mL, 20 ng/mL, 20 ng/mL, and 10 ng/mL, respectively. In this way, the culture medium (1) was obtained.

[Preparation method for culture medium (3)]

"Stem Fit AK02N solution A" (400 mL) manufactured by Ajinomoto Corporation, "Stem Fit AK02N solution B" (100 mL) manufactured by Ajinomoto Corporation, and "Stem Fit AK02N solution C" (2 mL) manufactured by Ajinomoto Corporation were mixed. In this way, the culture medium (3) was obtained.

In Examples 1 to 3 and Comparative Examples 1 to 3 below, cell culture and introduction of a reprogramming factor using a sendaiviral vector were performed according to the protocol disclosed in Non-Patent Document 1 except for the parts particularly described below.

(Example 1)

Step (1):

As somatic cells, blood-derived mononuclear cells were prepared. In addition, 1.5 mL of the culture medium (1) per 1 well was added to the culture vessel (S). Subsequently, mononuclear cells of $1.0 \times 10^6$ cells/well were seeded in the culture vessel (S), and cultured in an incubator at 37°C and a $CO_2$ concentration of 5% for 5 days. On Day 3 from the start of the culture, 0.75 mL of the liquid in the culture vessel was replaced with 0.75 mL of a fresh liquid culture medium.

Step (2):

Somatic cells after 5 days of culture in the step (1) were collected in a 15 mL tube. Then, the collected somatic cells were housed in a 1.5 mL tube with the number of cells of $3 \times 10^5$ cells, and the 1.5 mL tube was centrifuged under the conditions of $300 \times g$ and 5 minutes. After centrifugation, a supernatant was removed, and an appropriate amount of the culture medium (2) was added to a 1.5 mL tube. Then, to the 1.5 mL tube, a sendaiviral vector ("SRV™ IPSC-4 Vector" manufactured by Tokowa Bio Co., Ltd.) was added so that the MOI = 3. Then, the 1.5 mL tube was allowed to stand in a 37°C incubator for 2 hours. Next, 1 mL of the culture medium (2) was added to a 1.5 mL tube, and then centrifuged under the conditions of $300 \times g$ and 5 minutes. After centrifugation, the supernatant was removed and tapped. The steps of addition of the culture medium (2), centrifugation, and removal of the supernatant described above were repeated two more times. Next, 0.1 mL of the culture medium (2) was added to a 1.5 mL tube and mixed well. In this way, the reprogramming factor was introduced into the somatic cells.

After introducing the reprogramming factor into the somatic cells, the step (3) was performed.

Step (3):

To the culture vessel (S) was added 0.75 mL of the culture medium (2) per well. The somatic cells into which the reprogramming factor obtained in the step (2) was introduced were seeded on the cell scaffold in the culture vessel (S) at a cell number of $1.5 \times 10^5$ cells/well, and subjected to adhesion culture in an incubator at 37°C and a $CO_2$ concentration of 5%. On Days 1, 3, 5, and 7 from the start of the culture in the step (3), 0.5 mL of the fresh culture medium (3) was added to the culture vessel (S). From Day 9 after the start of the culture in the step (3), the liquid in the culture vessel (S) was replaced with 0.75 mL of the fresh culture medium (3) at a frequency of once or more every two days. When the colony morphology was confirmed on Day 14 after the start of culture in the step (3), it was a colony morphology of the iPS cells.

(Examples 2 and 3 and Comparative Examples 1 to 3)

Steps (1) to (3) were performed in the same manner as in Example 1 except that the kind of the culture vessel used in the step (1) or (3) was changed as shown in the following table. In Examples 2 and 3 and Comparative Examples 1 to 3, when the colony morphology was confirmed on Day 14 from the start of culture in the step (3), the colony morphology was a colony morphology of iPS cells.

(Evaluation)

In the following description and drawings, the number used together with P means the number of passages from the start of the step (3). P0 is passage 0, and is specifically the somatic cells obtained in the step (2) (the somatic cells seeded in the culture vessel at the start of the step (3)). The cells were passaged as follows.

Passages of cells:

The cell population on Day 14 from the start of the step (3) was made into single cells using a detachment agent ("TrypLE Select" manufactured by Thermo Fisher Scientific). From the obtained single cells, the total number of cells present in each well of the cell culture vessel was calculated by a fully automated cell counter. In addition, cells were passaged by seeding $1 \times 10^4$ cells to $50 \times 10^4$ cells in the same type of the culture vessel based on the calculated total cell number. The cells were passaged once or twice a week, and the culture medium (3) was used as a culture medium. At the time of passage, a ROCK inhibitor (Y-27632) was added to the culture medium (3) so as to have a final concentration of 10 $\mu$mol/L. After the day after the passage, the culture medium was changed at a frequency of once or more for 3 days.

In the following description and drawings, the first letters of SS, LS, FS, LL, FL, and SL mean the type of the culture vessel used in the step (1), and the second letters thereof mean the type of the culture vessel used in the step (3). For example, LS means that the culture vessel (L) was used in the step (1) and the culture vessel (S) was used in the step (3).

(1) Residual rate of virus

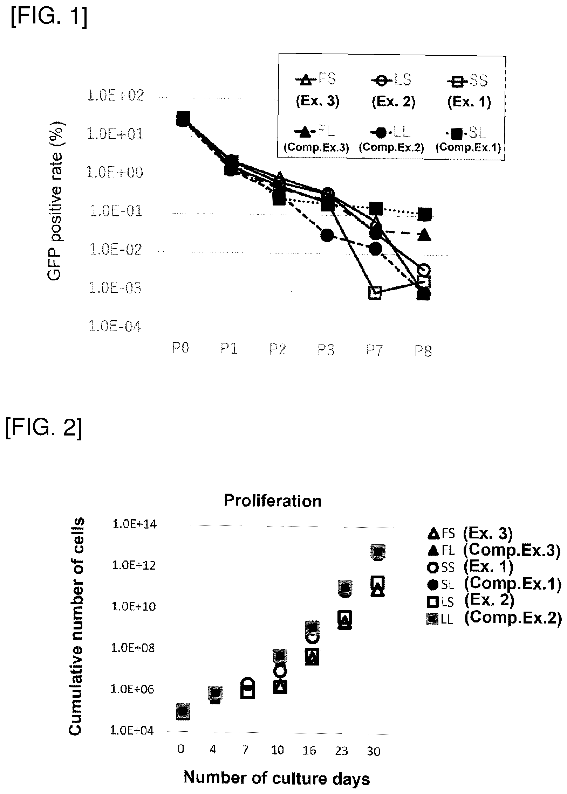

Some portions of the single cells were collected in 1.5 mL tubes at the time of passaging the cells. The collected single cells were measured by flow cytometry ("Attune NxT Flow Cytometer" manufactured by Thermo Fisher Scientific). The percentage of GFP-positive cells contained in the main cell population developed in the FSC-SSC dot plot was analyzed. The results are illustrated in Fig. 1. The smaller the GFP positive rate on the vertical axis in Fig. 1, the smaller the residual rate of virus. As illustrated in Fig. 1, Example 1 was particularly excellent in the result of the residual rate of virus.

(2) Cell proliferation and cell viability

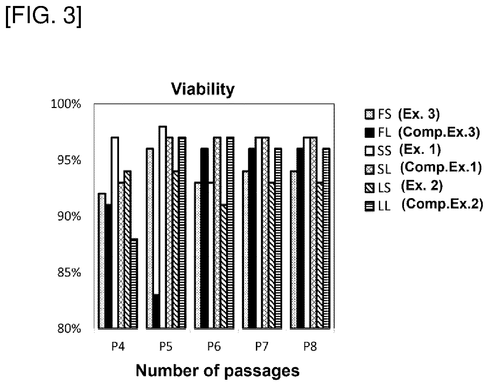

According to the number of days of culture from the start of the step (3), the cell proliferation and the cell viability were evaluated by the following procedure. The cells collected at the time of passage were counted in a fully automated cell counter. The number of cells in the well and the cell viability were calculated from the obtained results. In addition, the number of cells in the well was divided by the number of seeded cells to calculate the cell proliferation rate during that period, and the theoretical cumulative number of cells obtained when the total number of cells was passaged during the evaluation period was calculated. The results are illustrated in Figs. 2 and 3. Fig. 2 is a diagram illustrating the relationship between the number of culture days after the somatic cells into which the reprogramming factor has been introduced are cultured (the number of culture days after the start of the step (3)) and the cumulative number of cells. Fig. 3 is a diagram illustrating the relationship between the number of passages after the somatic cell into which the reprogramming factor has been introduced is cultured (the number of passages after the step (3) is started) and the viability of the cell. As illustrated in Figs. 2 and 3, the results of cell proliferation of Example 1 were equivalent to the results of cell proliferation of Comparative Examples 1 to 3. The results of the cell viability of Examples 1 to 3 were equivalent to the results of the cell viability of Comparative Examples 1 to 3.

(3) Doubling time

The doubling time of the cells from the time of cell collection of P5 to the time of cell collection of P8 (average time required for doubling the number of cells) was calculated by the following procedure. First, the cumulative number of cells at the time of cell collection of P8 determined in the above "(2) Cell proliferation and cell viability" was divided by the cumulative number of cells at the time of cell collection of P5 to calculate a cell proliferation ratio. The doubling time was calculated from the calculated cell proliferation ratio and the culture time (1 day =24 hours). The results are shown in the following table. As shown in the table, Example 1 was particularly excellent in the result of the doubling time.

(4) Undifferentiated (cell surface markers)

(4-1) Microscopic observation

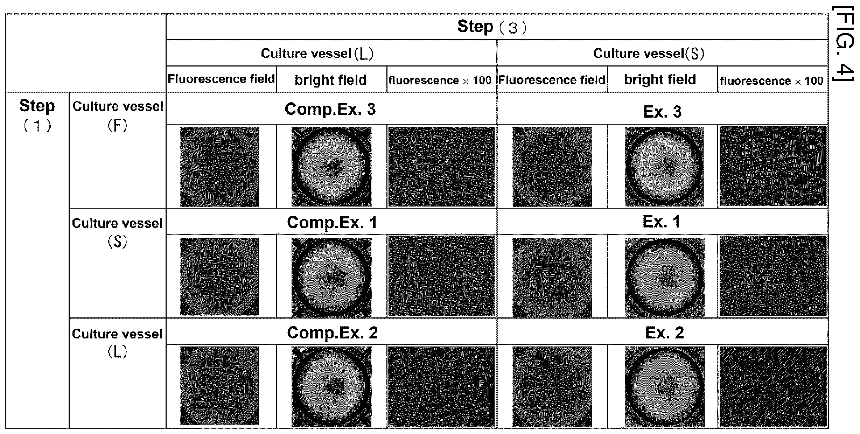

Using the cells of P3 during the culture, undifferentiated markers on the cell surface were stained by the following procedure, and undifferentiation was evaluated. An antibody against the TRA-1-60, which was labeled with a NL557 fluorescent dye ("Glo LIVE anti TRA-1-60 NL557" manufactured by R&D Systems, Inc.) was prepared. In addition, an antibody containing culture medium (3) was prepared by diluting this antibody 50 times with the culture medium (3). The cells of P3 during the culture were seeded in the culture vessel used in the step (3), and on Day 6 from the seeding, the culture medium (3) in the culture vessel was replaced with the antibody-containing culture medium (3). Then, the cells were cultured in an incubator at 37°C and a $CO_2$ concentration of 5% for 1 hour. After 1 hour of culture, the wells of the culture vessel were washed once with $1 \times$ DPBS. Using a fluorescence microscope ("BZ-X800" manufactured by KEYENCE CORPORATION), the well was observed in a fluorescence field or a bright field, and photographed. The results are illustrated in Fig. 4. In Fig. 4, the fluorescence field and the bright field are imaging results at a magnification of 20 times, and fluorescence $\times$ 100 is imaging results at a magnification of 100 times. Note that the images of the fluorescence field and the bright field in Fig. 4 are images obtained by acquiring the entire field of view in the well by overall batch automatic photographing and combining the entire well into one with image software manufactured by Keyence Corporation. In Examples 1 to 3 and Comparative Examples 1 to 3, there was no large difference in the area occupied by the cells in the well. On the other hand, in Examples 1 to 3, the number of cells labeled with the antibody was larger than that in Comparative Examples 1 to 3. In Examples 1 to 3, the cells were more brightly labeled as compared to Comparative Examples 1 to 3, and particularly in Example 1, the cells were more brightly labeled as compared to Examples 2 and 3.

(4-2) Flow cytometry measurement

An antibody against TRA-1-60, which was labeled with a PE fluorescent dye ("TRA-1-60 PE Antibody" manufactured by Thermo Fisher Scientific) was prepared. In addition, an antibody against SSEA4 labeled with a PE fluorescent dye ("SSEA4 PE Antibody" manufactured by Thermo Fisher Scientific) was prepared. P6 cells were contacted with these antibodies. Next, the positive rate of TRA-1-60 and its MFI, and the positive rate of SSEA4 and its MFI were determined using flow cytometry ("Attune NxT Flow Cytometer" manufactured by Thermo Fisher Scientific). The MFI is median fluorescence intensity. Specifically, evaluation was performed by the following procedure.

The P6 cells were made into single cells using a detachment agent ("TrypLE Select" manufactured by Thermo Fisher Scientific) in the same manner as in the "passage of cells" described above. The obtained single cells were subjected to a fully automated cell counter, and the cell concentration was calculated. Then, $5 \times 10^5$ cells were collected in a 1.5 mL tube, and the 1.5 mL tube was centrifuged at $300 \times$ g for 5 minutes. After centrifugation, the supernatant was removed and $1 \times$ DPBS was added to a 1.5 mL tube. Subsequently, centrifugation and removal of the supernatant were performed again under the above conditions. Blocking was then performed in 1 wt% BSA-containing DPBS. Next, an antibody solution containing the above-described two kinds of antibodies ("TRA-1-60 PE Antibody" manufactured by Thermo Fisher Scientific and "SSEA4 PE Antibody" manufactured by Thermo Fisher Scientific) in appropriate amounts was added to a 1.5 mL tube, and the tube was incubated in a dark place and at room temperature for 1 hour. The 1.5 mL tube was then centrifuged at $300 \times$ g for 5 minutes before the supernatant was removed and washed twice with $1 \times$ DPBS. Next, 0.4 mL of PBS containing 1% by weight BSA was added to a 1.5 mL tube, mixed, and measured by flow cytometry. The proportion of TRA-1-60 positive cells and the proportion of SSEA4 positive cells included in the main cell population developed in the FSC-SSC dot plot were analyzed. The results are shown in the following table.

(5) Undifferentiated (gene expression marker)

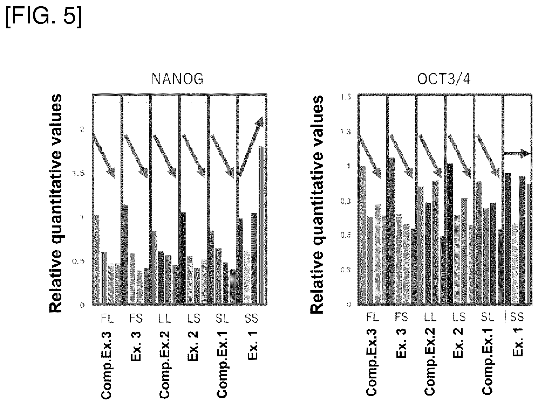

Using the cells of P0, P1, P3, and P7, relative quantification of undifferentiated marker genes expressed in each cell was performed. Relative quantification was performed using a PCR apparatus ("QuatnStudio3 Real-Time PCR System" manufactured by Thermo Fisher Scientific). Specifically, evaluation was performed by the following procedure. At the time of passage, the cell population was detached from the culture vessel to singulate, and $2 \times 10^5$ cells were collected in a 1.5 mL tube. Next, cell lysis, cDNA synthesis, and quantitative PCR were performed using "TaqMan Fast Advanced Cells-to-Ct Kit" manufactured by Thermo Fisher Scientific. The target gene expression markers were NANOG and OCT3/4, and "TaqMan Gene Expression Assay" manufactured by Thermo Fisher Scientific was used as each primer and detection reagent. The relative quantitative value was calculated based on the gene amount of P0 cells in FL. The results are illustrated in Fig. 5. Fig. 5 illustrates results in the order of P0, P1, P3, and P7 from the left side to the right side.

(6) Differentiation potency (formation of embryoid body)

(6-1) Microscopic observation

Differentiation induction of iPS cells:

A 96-well plates ("ultra-low adsorption round bottom 96 well plates" manufactured by Corning Incorporated) was prepared. In addition, "Essential 6" manufactured by Thermo Fisher Scientific was prepared as a culture medium. The culture medium was added to the wells of the 96-well plate. A ROCK inhibitor (Y-27632) was previously added to the culture medium to be used at the time of seeding cells so as to have a final concentration of 10 $\mu$mol/L. The P3 cells (iPS cells, single cells) cultured in the step (3) were seeded at a cell number of $1.5 \times 10^4$ cells/well, centrifuged at $300 \times g$ for 5 minutes, and then cultured in suspension in an incubator at 37°C and a $CO_2$ concentration of 5%. The culture medium replacement was performed the day after seeding and every other day thereafter.

Microscopic observation:

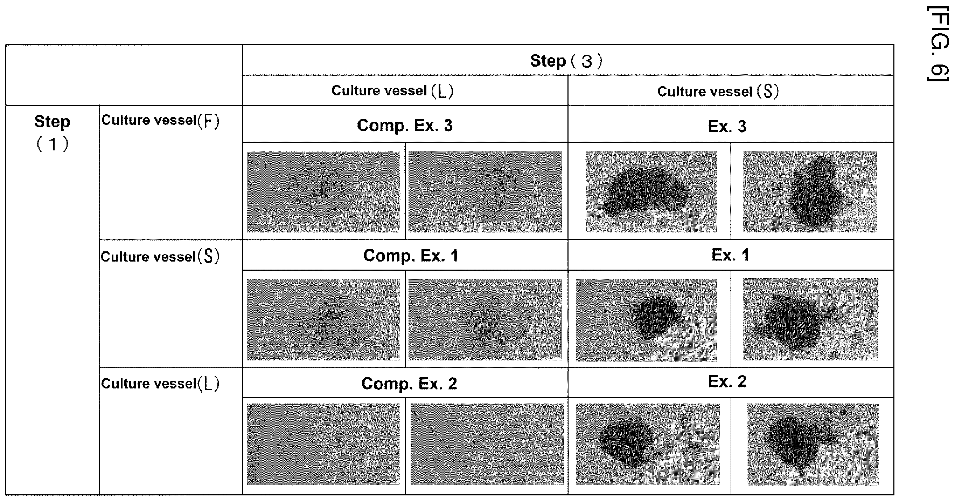

Until Day 39 after seeding, the cultured cells were observed under a microscope. The appearance of the cells on Day 39 (n = 2) is illustrated in Fig. 6. In Examples 1 to 3, cell aggregation and formation of embryoid body were observed. On the other hand, in Comparative Examples 1 to 3, the formation of embryoid body was not observed.

(6-2) Gene expression (qPCR)

Triderm markers in the embryoid body obtained in Examples 1 to 3 were evaluated as follows. Single cells at the time of seeding (cells at the start of culture (Day 0)), cells (embryoid bodies) at Day 11 from the start of culture, cells (embryoid bodies) at Day 18 from the start of culture, and cells (embryoid bodies) at Day 39 from the start of culture were collected, and these embryoid bodies were treated with an enzyme-containing solution for cell detachment (Accumax) to obtain single cells. Next, cell lysis, cDNA synthesis, and quantitative PCR were performed using "TaqMan Fast Advanced Cells-to-Ct Kit" manufactured by Thermo Fisher Scientific. As gene expression markers of the target, AFP, FOXA2, GATA4, GATA6, SOX17, and SOX7 were set as endoderm markers, KDR and T were set as mesoderm markers, and NES, PAX6, and SOX1 were set as ectoderm markers. "TaqMan Gene Expression Assay" manufactured by Thermo Fisher Scientific was used as each primer and detection reagent. The relative quantitative value was calculated based on the gene amount of P0 cells for each gene expression.

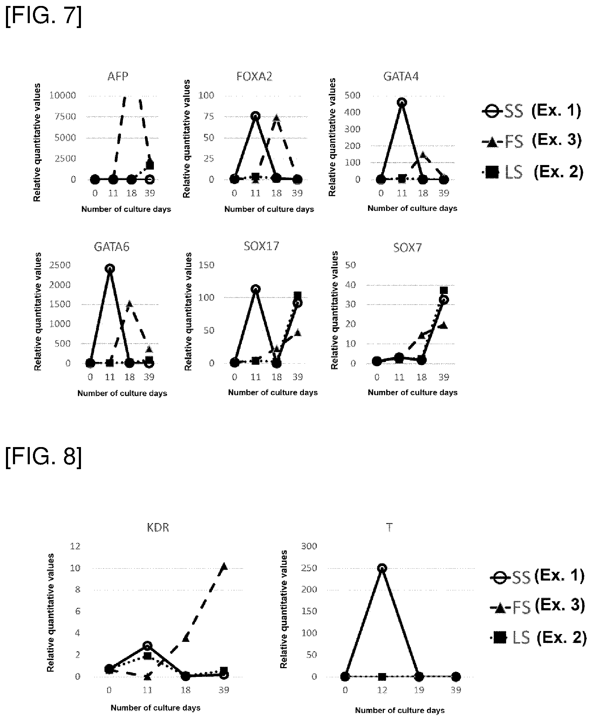

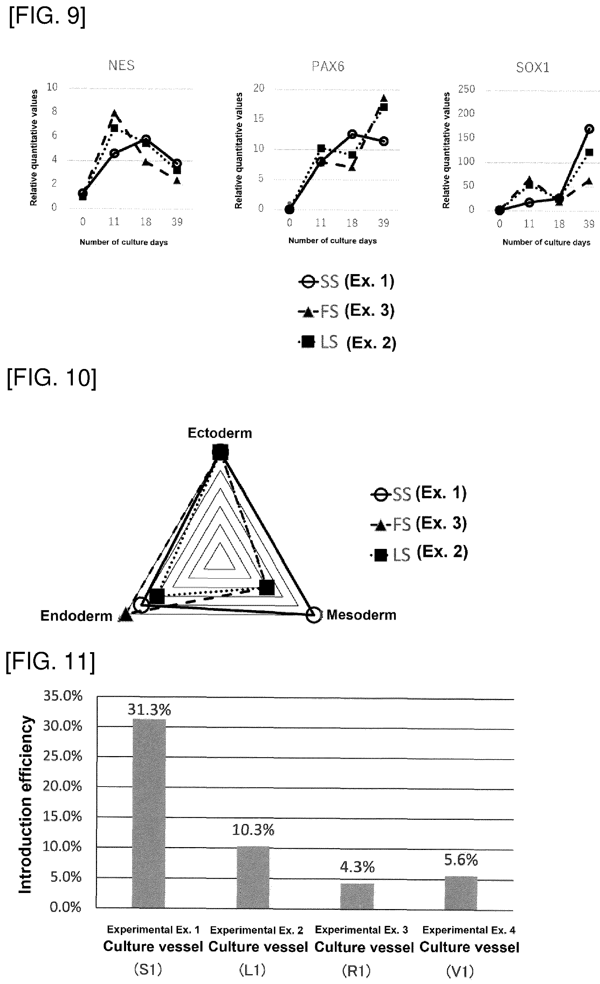

The results of evaluating the gene expression of each marker described above by qPCR are illustrated in Figs. 7 to 9. In addition, Fig. 10 illustrates a radar chart summarizing the differentiation score into each germ layer by scoring the presence or absence of an increase in the marker. Scores are evaluated for endoderm, mesoderm, and ectoderm as 1, 3, and 2 for an increase in one marker, respectively.

The results are shown in Table 1 below.

[Table 1]

| | | | | Example 1 | Example 2 | Example 3 | Comparative Example 1 | Comparative Example 2 | Comparative Example 3 |
|---|---|---|---|---|---|---|---|---|---|
| Step (1) | Type of culture vessel | | | S | L | F | S | L | F |
| Step (3) | Type of culture vessel | | | S | S | S | L | L | L |
| Evaluation | (1) Residual rate of virus | | | Fig. 1 | | | | | |
| | (2) Cell proliferation and cell viability | Proliferation | | Fig. 2 | | | | | |
| | | Viability | | Fig. 3 | | | | | |
| | (3) Doubling time | | Hours | 25.2 | 28.2 | 30.6 | 27.9 | 28.3 | 27.1 |
| | (4) Undifferentiated (Cell surface marker) | (4-1) Microscopic observation | | Fig. 4 | | | | | |
| | | (4-2) Flow cytometry measurement | TRA-1-60 | Positive rate (%) | 94.5 | 88.3 | 89.0 | 73.0 | 79.2 | 77.0 |
| | | | | MFI | 1022 | 729 | 770 | 449 | 508 | 486 |
| | | | SSEA4 | Positive rate (%) | 99.4 | 99.1 | 99.2 | 99.5 | 99.1 | 99.4 |
| | | | | MFI | 9522 | 9054 | 10310 | 10355 | 9397 | 10489 |
| | (5) Undifferentiated (gene expression marker) | | | Fig. 5 | | | | | |
| | (6) Differentiation potency (Formation of embryoid body) | (6-1) Microscopic observation | | Fig. 6 | | | | | |
| | | (6-2) Gene expression (qPCR) | | Figs. 7 to 10 | | | - | - | - |

EP 4 589 004 A1

16

In addition, as Experimental Examples 1 to 4, an experiment of introducing the following mRNA into somatic cells was performed.

The following were prepared as culture vessels.

Culture vessel (S1) (a plate provided with a cell scaffold containing a peptide-conjugated polyvinyl acetal resin, produced by the following production method (S1))

Culture vessel (L1) (a plate coated with "Laminin 511E8 fragment (iMatrix-511)" manufactured by Nippi. Inc., "mircroplate with a lid, Flat bottom, TC treated polystylene" manufactured by IWAKI)

Culture vessel (R1) (a plate coated with "Retronectin (T100A)" manufactured by Takara Bio Inc., "Falcon cell culture 48-well multiwell plate (353078)" manufactured by Corning Incorporated)

Culture vessel (V1) (a plate coated with "Vitronectin (220-02041)" manufactured by FUJIFILM Wako Pure Chemical Corporation, "Falcon cell culture 48-well multiwell plate (353078)" manufactured by Corning, Incorporated)

[Preparation method for culture vessel (S1)]

A culture vessel (S1) was obtained in the same manner as in the above-mentioned "preparation method for culture vessel (S)" except that 10 $\mu$L of the coating liquid was applied to each well of a 48-well plate (bottom area of each well: 0.75 cm$^2$) by a cast method.

(Experimental Example 1)

Introduction of mRNA-eGFP into somatic cells:

Human peripheral blood CD8+T cells ("2W-300" manufactured by Lonza) were prepared as somatic cells. In addition, 0.2 mL of the culture medium (1) per well was added to the culture vessel (S1). Next, $1.4 \times 10^5$ cells/well of CD8+T cells were seeded in a culture vessel (S1), and cultured in an incubator at 37°C and a $CO_2$ concentration of 5% for 2 days. Next, 2 $\mu$g/well of mRNA-eGFP ("CleanCap EGFP mRNA" manufactured by TriLink BioTechnologies, Inc.) was added to the culture vessel (S1), and mRNA-eGFP was introduced into CD8+T cells by a plasma gene introduction method. The plasma irradiation conditions were as follows.

Power supply frequency: 50 kHz
Discharge voltage: 16 kV$_{p-p}$
Discharge time: 30 msec
Distance between electrode end and upper surface of culture vessel: 1 mm

Next, 0.2 mL of TransAct (manufactured by Milteny) per well was added to the culture vessel (S1) and cultured for 30 hours.

Evaluation of introduction efficiency (flow cytometry measurement) :

The cells after culturing for 30 hours were peeled off from the culture vessel by pipetting to obtain single cells. The obtained single cells were measured using flow cytometry ("Attune NxT Flow Cytometer" manufactured by Thermo Fisher Scientific), and the number of living cells (R1) and the number of cells having GPP fluorescence (R2) were determined. The introduction efficiency of mRNA-eGFP was calculated by the following formula.

$$\text{Introduction efficiency (\%) = (R2/R1)} \times 100$$

R1: Number of living cells
R2: Number of cells with GFP fluorescence

(Experimental Examples 2 to 4)

The introduction efficiency of mRNA-eGFP was calculated in the same manner as in Experimental Example 1 except that the type of culture vessel was changed as illustrated in Fig. 11.

(Results)

As illustrated in Fig. 11, when the culture vessel (S1) was used, the introduction efficiency of mRNA-eGFP into somatic cells was higher than that when the culture vessels (L1), (V1), and (R1) were used. That is, in the presence of the cell scaffold containing the peptide-conjugated polyvinyl acetal resin, the introduction efficiency of mRNA into the somatic cells was able to be further enhanced as compared with the presence of the cell scaffold containing a protein.

In addition, as Example 4, an establishment test for iPS cells using the following mRNAs was performed.

(Example 4)

Step (1):

Peripheral blood mononuclear cells ("Human PBMC 10 M Healthy Japanese (Cat#: 93210-10M)" manufactured by Precision for medicine) were prepared as somatic cells. In addition, 0.4 mL of the culture medium (1) per well was added to the culture vessel (S1). Next, peripheral blood mononuclear cells of $5.0 \times 10^5$ cells/well were seeded in a culture vessel (S1), and cultured in an incubator at 37°C and a $CO_2$ concentration of 5% for 5 days. On Day 3 from the start of culture, 0.2 mL of the culture medium (1) in the culture vessel (S1) was replaced with 0.2 mL of fresh culture medium (1).

Step (2):

The culture medium (1) was removed from the culture vessel (S1) after 5 days of culture in the step (1). Next, 0.8 $\mu$g/well of mRNA ("ReproRNA-OKSGM" manufactured by STEMCELL Technologies, C/N: ST-05931) was added to the culture vessel (S1), and mRNA was introduced into peripheral blood mononuclear cells by a plasma gene introduction method. The plasma irradiation conditions were as follows.

Power supply frequency: 50 kHz
Discharge voltage: 16 $kV_{p-p}$
Discharge time: 30 msec
Distance between electrode end and upper surface of culture vessel: 1 mm

Step (3):

The following culture media (3A) and (3B) were prepared.

Culture medium (3A):

Recombinant B18R Protein (manufactured by STEMCELL Technologies) was added in an amount of 0.175 $\mu$g to 1 mL of the culture medium (1). In this way, a culture medium (3A) was obtained.

Culture medium (3B):

Recombinant B18R Protein (manufactured by STEMCELL Technologies) was added in an amount of 0.175 $\mu$g to 1 mL of the culture medium (3). In this way, a culture medium (3B) was obtained.

To the culture vessel (S1), 0.2 mL of the culture medium (3A) per well was added, and somatic cells into which mRNA (reprogramming factor) had been introduced were adhesively cultured in an incubator at 37°C and a $CO_2$ concentration of 5% for 1 day. After adhesion culture for 1 day, 0.133 mL of the culture medium (3B) was added per well, and the cells were cultured in an incubator at 37°C and a $CO_2$ concentration of 5%. From the start of culture in the step (3) to Day 8, 0.135 mL of the culture medium (3B) was added per well every 2 to 3 days, and from Day 9 after the start of culture in the step (3), the whole amount of the culture medium (3B) was replaced with fresh culture medium (3B) every 2 to 3 days.

(Evaluation)

(1) Microscopic observation

An antibody against the TRA-1-60, which was labeled with a NL557 fluorescent dye ("Human Glo LIVE anti TRA-1-60 (R) Northern Lights NL557-conjugated Antibody" manufactured by R&D Systems, Inc.) was prepared. In addition, an antibody-containing culture medium (3B) obtained by diluting this antibody 50 times with the culture medium (3B) was prepared. On Day 35 from the start of the culture in the step (3), the culture medium (3B) in the culture vessel (S1) was replaced with the antibody-containing culture medium (3B). Then, the cells were cultured in an incubator at 37°C and a $CO_2$ concentration of 5% for 30 minutes. After 30 minutes of culture, the wells of the culture vessel (S1) were washed with PBS and replaced with a new culture medium (3B). Using a fluorescence microscope ("BZ-X800" manufactured by KEYENCE CORPORATION), the well was observed in a fluorescence field or a bright field, and photographed. As a result, the colony morphology of the iPS cells was observed, and many cells labeled with an antibody were also observed.

(Example 5)

As culture vessels, the culture vessel (S1) and the culture vessel (L1) described above and the following culture vessels (S2) and (S3) were prepared.

Culture vessel (S2) (a plate provided with a cell scaffold containing a peptide-conjugated polyvinyl acetal resin, produced by the following production method (S2))
Culture vessel (S3) (a plate provided with a cell scaffold containing a peptide-conjugated polyvinyl acetal resin, produced by the following production method (S3))

[Preparation method for culture vessel (S2)]

A culture vessel (S2) was obtained in the same manner as in the above-mentioned "preparation method for culture vessel (S)" except that 10 μL of the coating liquid was applied to each well of a 96-well plate (bottom area of each well: 0.32 cm$^2$) by a cast method.

[Preparation method for culture vessel (S3)]

A culture vessel (S3) was obtained in the same manner as in the above-mentioned "preparation method for culture vessel (S)" except that 100 μL of the coating liquid was applied to each well of a 12-well plate (bottom area of each well: 3.8 cm$^2$) by a cast method.
The following culture media (4A), (4A+), (4B), and (4B+) were prepared.

Culture medium (4A):

"Fibroblast growth medium: Fibroblast Growth Medium (Ready-to-use)" manufactured by Takara Bio Inc. was used as a culture medium (4A).

Culture medium (4A+):

Recombinant B18R Protein (manufactured by STEMCELL Technologies) was added in an amount of 0.175 μg to 1 mL of the culture medium (4A), and a penicillin-streptomycin mixed solution (manufactured by Nacalai Tesque) was added in an amount of 10 μL. In this way, the culture medium (4A+) was obtained.

Culture medium (4B):

"StemFit AK02N" manufactured by Ajinomoto Corporation was used as a culture medium (4B).

Culture medium (4B+):

Recombinant B18R Protein (manufactured by STEMCELL Technologies) was added in an amount of 0.175 μg to 1 mL of the culture medium (4B), and a penicillin-streptomycin mixed solution (manufactured by Nacalai Tesque) was added in an amount of 10 μL. In this way, the culture medium (4A+) was obtained.

Step (1):

Human fibroblasts ("normal human skin fibroblasts (children)" manufactured by Takara Bio Inc.) were prepared as somatic cells. In addition, 0.2 mL of the culture medium (4A) per well was added to the culture vessel (S1). Next, human fibroblasts of $1.0 \times 10^3$ cells/well were seeded in the culture vessel (S1), and cultured in an incubator at 37°C and a $CO_2$ concentration of 5% for 6 days. On Day 3 from the start of the culture, 0.2 mL of the culture medium (4A) in the culture vessel (S1) was replaced with 0.2 mL of the fresh culture medium (4A).

Step (2):

The culture medium (4A) was removed from the culture vessel (S1) after culturing for 6 days in the step (1). Next, 0.8 μg/well of mRNA ("ReproRNA-OKSGM" manufactured by STEMCELL Technologies, C/N: ST-05931) was added to the culture vessel (S1), and mRNA was introduced into human fibroblasts by a plasma gene introduction method. The plasma irradiation conditions were as follows.
Power supply frequency: 50 kHz
Discharge voltage: 16 kV$_{p-p}$
Discharge time: 30 msec
Distance between electrode end and upper surface of culture vessel: 1 mm

Step (3):

To the culture vessel (S1), 0.4 mL of the culture medium (4A+) per well was added, and somatic cells into which mRNA (reprogramming factor) had been introduced were adhesively cultured in an incubator at 37°C and a CO$_2$ concentration of 5%. After 1 day of culture, the total volume of culture medium was replaced with 0.4 mL of fresh culture medium (4A+). On Day 4 from the start of the culture in the step (3), the total volume of culture medium was replaced with fresh culture medium (4A+) containing 0.8 μg Puromycin per 1 mL.
On Day 7 from the start of the culture in the step (3), the entire amount of the culture medium was replaced with fresh culture medium (4B+). Thereafter, until Day 14 from the start of culture in the step (3), the whole amount of the culture medium was replaced with fresh culture medium (4B+) every three days.
On Day 14 from the start of the culture in the step (3), the number of colonies of iPS cells was confirmed. Colonies were peeled off by pipetting and collected in a culture vessel (S2) (P1). To the culture vessel (S2), 0.1 mL of the culture medium (4B) was added per well, and the cells were cultured in an incubator at 37°C and a CO$_2$ concentration of 5%. The total volume of culture media was replaced with fresh culture medium (4B) every second day. On Day 6 from the start of culture in the culture vessel (S2), single cells were obtained using a detachment agent ("TrypLE Select" manufactured by Thermo Fisher Scientific). From the obtained single cells, $2 \times 10^4$ cells were seeded in a culture vessel (S3) to passage the cells (P2). The cells were passaged once a week, and the culture medium (4B) was used as a culture medium (P3 to P7). At the time of passage, a ROCK inhibitor (Y-27632) was added to the culture medium (4B) so as to have a final concentration of 10 μmol/L. In addition, after the day following the passage, the culture medium was replaced at a frequency of once every two days.

(Comparative Example 4)

Steps (1) to (3) on Day 14 were performed in the same manner as in Example 5 except that the culture vessel (L1) was used instead of the culture vessel (S1).

(Evaluation)

(1) Undifferentiated (cell surface markers)

(1-1) Microscopic observation

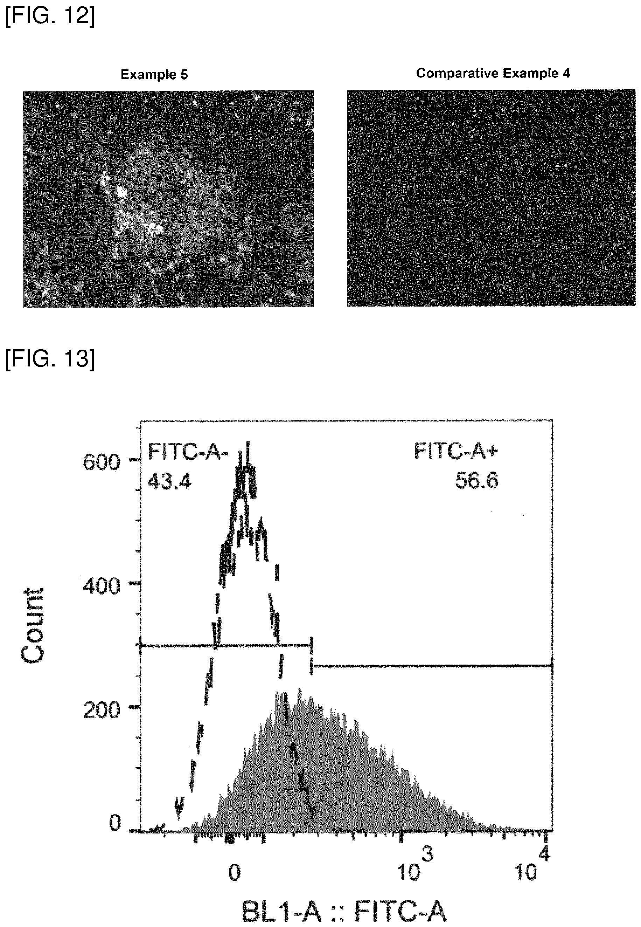

Using cells on Day 14 after plasma irradiation, undifferentiated marker on the cell surface was stained by the following procedure, and undifferentiation was evaluated. An antibody against the TRA-1-60, which was labeled with a NL557 fluorescent dye ("Glo LIVE anti TRA-1-60 NL557" manufactured by R&D Systems, Inc.) was prepared. In addition, an antibody-containing culture medium was prepared by diluting this antibody 50 times with the culture medium (4B). The culture medium (4B) in the culture vessel was replaced with the antibody-containing culture medium, and cultured in an incubator at 37°C and a CO$_2$ concentration of 5% for 1 hour. After 1 hour of culture, the wells of the culture vessel were washed once with 1 × DPBS. The culture vessel was observed and photographed in a fluorescence field using a fluorescence microscope ("BZ-X800" manufactured by KEYENCE CORPORATION). The results are illustrated in Fig. 12. Fig. 12 is a photographing result of the fluorescence field at a magnification of 100 times.
As illustrated in Fig. 12, in Example 5, cells labeled with the antibody (TRA-1-60 positive cells) were favorably obtained. On the other hand, in Comparative Example 4, TRA-1-60 positive cells were hardly obtained. Therefore, the subsequent evaluation was performed only on the cells obtained in Example 5.

(1-2) Flow cytometry measurement

An antibody against the TRA-1-60, which was labeled with a FITC fluorescent dye ("TRA-1-60 FITC Antibody" manufactured by Becton, Dickinson and Company) was prepared. Cells at Day 7 of P3 were reacted with this antibody and expression of TRA-1-60 was analyzed using flow cytometry ("Attune NxT Flow Cytometer" manufactured by Thermo Fisher Scientific). Specifically, evaluation was performed by the following procedure.

Cells at Day 7 of P3 were single cells using a detachment agent ("TrypLE Select" manufactured by Thermo Fisher Scientific) in the same manner as in the "passage of cells" procedure described above. The obtained single cells $5 \times 10^5$ cells were collected in a 1.5 mL tube, and the 1.5 mL tube was centrifuged at $300 \times$ g for 5 minutes. After centrifugation, the supernatant was removed, and Pharmingen Stain Buffer (FBS) manufactured by BD was added to a 1.5 mL tube. Subsequently, centrifugation and removal of the supernatant were performed again under the above conditions. Next, blocking was performed with Pharmingen Stain Buffer (FBS) manufactured by Becton, Dickinson and Company. Next, a solution prepared by diluting 10 μL of the above-described antibody with 100 μL of Pharmingen Stain Buffer (FBS) manufactured by Becton, Dickinson and Company was added to a 1.5 mL tube, and the mixture was incubated in a dark place and at room temperature for 1 hour. Then, the 1.5 mL tube was centrifuged at $300 \times$ g for 5 minutes, and then the supernatant was removed and washed twice with Pharmingen Stain Buffer (FBS). Next, 0.4 mL of Pharmingen Stain Buffer (FBS) was added to a 1.5 mL tube, mixed, and measured by flow cytometry. A histogram plot of the fluorescence intensity of the FITC fluorescent dye is illustrated in Fig. 13.

In Fig. 13, the horizontal axis represents the fluorescence intensity of the FITC fluorescent dye, and the vertical axis represents the number of cells. The histogram plot (not colored) on the left side in Fig. 13 is a histogram plot when the cells were not reacted with the antibody, and the histogram plot (colored) on the right side in Fig. 13 is a histogram plot when the cells were reacted with the antibody. In addition, the maximum value of the fluorescence intensity of the histogram plot (not colored) on the left side in Fig. 13 was set as the boundary value between the negative region and the positive region in the histogram plot (colored) on the right side. In Fig. 13, the proportions (%) of the number of cells included in the negative region and the positive region in the right histogram plot (colored) are shown in "FITC-A-" and "FITC-A+", respectively.

(2) Undifferentiated (gene expression marker)

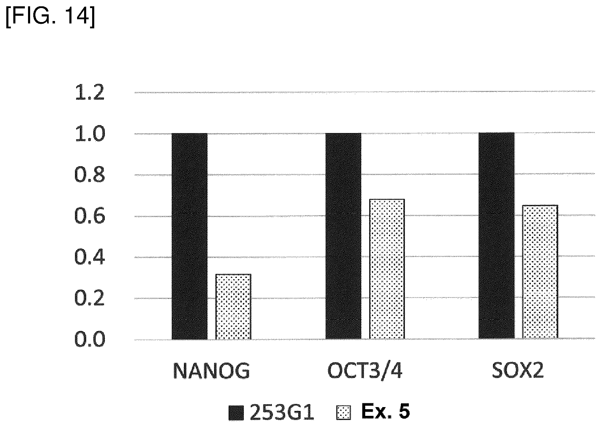

Relative quantification of undifferentiated marker genes expressed in each cell was performed using cells at Day 7 of P6. Relative quantification was performed using a PCR apparatus ("QuatnStudio3 Real-Time PCR System" manufactured by Thermo Fisher Scientific). Specifically, evaluation was performed by the following procedure. At the time of passage, the cell population was detached from the culture vessel to singulate, and $2 \times 10^5$ cells were collected in a 1.5 mL tube. Next, cell lysis, cDNA synthesis, and quantitative PCR were performed using "TaqMan Fast Advanced Cells-to-Ct Kit" manufactured by Thermo Fisher Scientific. The target gene expression markers were NANOG, OCT3/4, and SOX2, and "TaqMan Gene Expression Assay" manufactured by Thermo Fisher Scientific was used as each primer and detection reagent. The expression level of each gene expression marker was standardized by a ΔΔCT method using housekeeping gene 18S. The relative quantitative value was calculated with the expression level in the human fibroblast-derived iPS cells line (253G1) established at Kyoto University as 1. The results are illustrated in Fig. 14.

(3) Differentiation potency

iPS cell tridermal differentiation induction:

As a culture medium, each germ layer differentiation medium (Ectoderm Medium, Mesoderm Medium, Endoderm Medium) of "Trilineage Differentiation Kit" manufactured by Stemcell Technologies was prepared. The culture medium was added to each well of the culture vessel (S3). A ROCK inhibitor (Y-27632) was previously added to the culture medium to be used at the time of seeding cells so as to have a final concentration of 10 μmol/L. The cells at the time point of Day 7 of P7 were made into single cells using a detachment agent ("TrypLE Select" manufactured by Thermo Fisher Scientific) in the same manner as the procedure of "passage of cells" described above. Endodermal differentiation induction and ectodermal differentiation induction were seeded with the number of cells of $8 \times 10^5$ cells/well, and mesodermal differentiation induction was seeded with the number of cells of $2 \times 10^5$ cells/well, and centrifuged under the conditions of $300 \times$ g and 5 minutes, then adhesion culture was performed in an incubator under the conditions of 37°C and a $CO_2$ concentration of 5%. The culture medium was replaced once a day. As a comparison, tridermal differentiation induction was performed in the same manner using iPS cells line (253G1).

(3-1) Immunostaining

The following solutions and antibodies were prepared.

[Solvent]

Fixing solution: 4% paraformaldehyde
Blocking solution: 0.1% TritonX100, 10% FBS-containing PBS
Wash buffer: PBS containing 0.025% polyoxyethylene (20) sorbitan monolaurate

[Antibody]

Primary antibody (ectoderm): Anti-PAX6 antibody (Rabbit IgG)
Primary antibody (mesoderm): Anti-Brachyury/Bry antibody (Rabbit IgG H&L)
Primary antibody (endoderm): Anti-SOX17 antibody (Rabbit IgG)
Secondary antibody (Rabbit): Goat pAb to Rb IgG-Alexa Fluor488

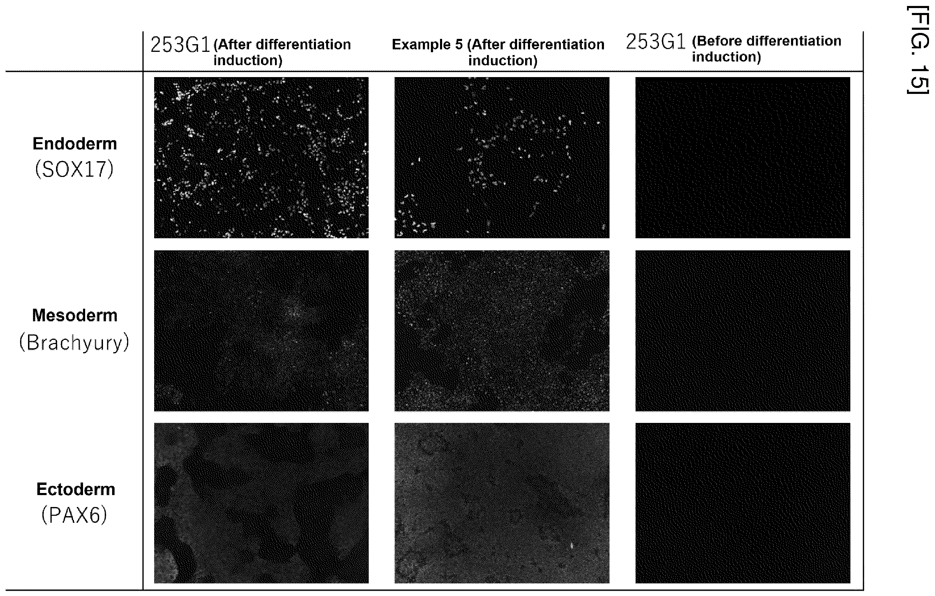

Immunostaining for endodermal differentiation induction and mesodermal differentiation induction was performed on Day 5 from the start of culture in each differentiation culture medium, and immunostaining for ectodermal differentiation induction was performed on Day 7 from the start of culture in the differentiation culture medium. First, the culture medium was removed from the culture vessel (S3), and then 0.75 mL of PBS was added thereto. Next, PBS was removed, 0.5 mL of a fixing solution was added, and the mixture was allowed to stand for 15 minutes. The fixing solution was removed and washed twice with 0.5 mL PBS. Thereafter, 500 μL of a blocking solution was added, and the resulting mixture was allowed to stand for 15 minutes. The blocking solution was removed, a Wash buffer was added, the mixture was allowed to stand for 5 minutes, and then the solution was removed. The Wash buffer was added again and allowed to stand for 5 minutes, and then the solution was removed. Thereafter, 300 μL/well of the primary antibody-containing solution was added, and the mixture was allowed to stand for 2 hours. Each primary antibody-containing solution was prepared by being diluted with a blocking solution. The primary antibody-containing solution was removed, 2 mL/well of the Wash buffer was added, and the mixture was allowed to stand for 5 minutes and then removed. The Wash buffer was added again and allowed to stand for 5 minutes to remove the solution. Thereafter, the secondary antibody-containing solution was added, and the mixture was incubated overnight in a refrigerator. The following day, staining was performed with DAPI diluted 1,000 fold with Wash buffer for 3 minutes. Washing was performed twice with 2 mL/well of Wash buffer. Next, the well was observed and photographed in a fluorescence field using a fluorescence microscope ("BZ-X800" manufactured by Keyence Corporation). An iPS cells line (253G1) before differentiation induction was used as a negative control. The results are illustrated in Fig. 15.

(3-2) Gene expression (qPCR)

On the day when the "(3-1) immunostaining" was performed, single cells were recovered using a detachment agent ("TrypLE Select" manufactured by Thermo Fisher Scientific) in the same manner as the procedure of the "passage of cells" described above. Next, cell lysis, cDNA synthesis, and quantitative PCR were performed using "TaqMan Fast Advanced Cells-to-Ct Kit" manufactured by Thermo Fisher Scientific. As gene expression markers of the target, FOXA2 and SOX17 were set as endoderm markers, T was set as a mesoderm marker, and PAX6 was set as an ectoderm marker. "TaqMan Gene Expression Assay" manufactured by Thermo Fisher Scientific was used as each primer and detection reagent. The expression level of each gene expression marker was standardized by a ΔΔCT method using housekeeping gene 18S. The iPS cells line (253G1) before differentiation induction and the cells at Day 7 of P7 were used as negative controls. The relative quantitative value was calculated with the expression level in the negative control of the iPS cells line (253G1) as 1 for each gene expression.

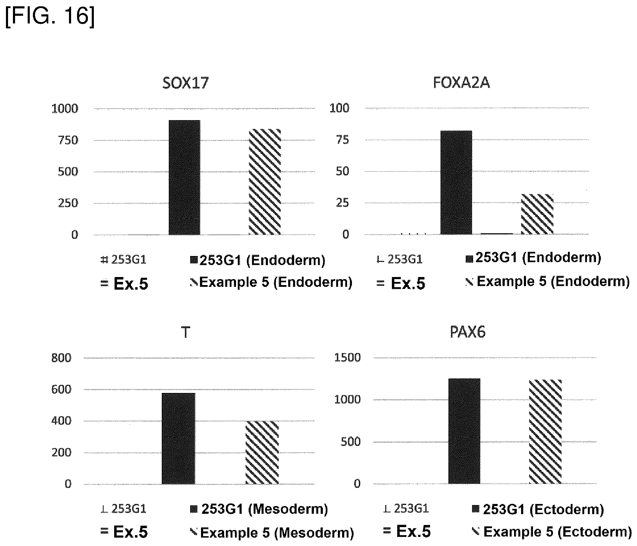

The results of evaluating the gene expression of each marker described above by qPCR are illustrated in Fig. 16. In Fig. 16, "253G1" and "Example 5" represent the results of the negative control, "253G1 (endoderm)" and "Example 5 (endoderm)" represent the results after endoderm differentiation induction, "253G1 (mesoderm)" and "Example 5 (mesoderm)" represent the results after mesoderm differentiation induction, and "253G1 (ectoderm)" and "Example 5 (ectoderm)" represent the results after ectoderm differentiation induction.

**Claims**

1. A production method for induced pluripotent stem cells, comprising the steps of:

   introducing a reprogramming factor into somatic cells; and
   culturing the somatic cells into which the reprogramming factor has been introduced in the presence of a cell scaffold containing a peptide-conjugated polyvinyl acetal resin.

2. The production method for induced pluripotent stem cells according to claim 1, further comprising:

   before the step of introducing the reprogramming factor into the somatic cells,
   a step of culturing somatic cells in the presence of a cell scaffold containing a peptide-conjugated polyvinyl acetal resin.

3. The production method for induced pluripotent stem cells according to claim 1 or 2, wherein in the step of introducing the reprogramming factor into the somatic cells, the reprogramming factor is introduced into the somatic cells using a sendaiviral vector.

4. The production method for induced pluripotent stem cells according to claim 1 or 2, wherein the reprogramming factor is mRNA.

5. The production method for induced pluripotent stem cells according to claim 4, wherein the mRNA is introduced into the somatic cells by a plasma gene introduction method.

[FIG. 1]

[FIG. 2]

[FIG. 3]

Viability

FS (Ex. 3)
FL (Comp.Ex.3)
SS (Ex. 1)
SL (Comp.Ex.1)
LS (Ex. 2)
LL (Comp.Ex.2)

Number of passages

[FIG. 4]

EP 4 589 004 A1

| | | Step (3) | | | | | |
|---|---|---|---|---|---|---|---|
| | | Culture vessel (L) | | | Culture vessel (S) | | |
| | | Fluorescence field | bright field | fluorescence × 100 | Fluorescence field | bright field | fluorescence × 100 |
| Step (1) | Culture vessel (F) | Comp.Ex. 3 | | | Ex. 3 | | |
| | | | | | | | |
| | Culture vessel (S) | Comp.Ex. 1 | | | Ex. 1 | | |
| | | | | | | | |
| | Culture vessel (L) | Comp.Ex. 2 | | | Ex. 2 | | |
| | | | | | | | |

[FIG. 5]

[FIG. 6]

EP 4 589 004 A1

| | | Step ( 3 ) | | | |
|---|---|---|---|---|---|
| | | Culture vessel (L) | | Culture vessel (S) | |
| Step ( 1 ) | Culture vessel(F) | Comp. Ex. 3 | | Ex. 3 | |
| | | | | | |
| | Culture vessel(S) | Comp. Ex. 1 | | Ex. 1 | |
| | | | | | |
| | Culture vessel(L) | Comp. Ex. 2 | | Ex. 2 | |
| | | | | | |

[FIG. 7]

[FIG. 8]

[FIG. 9]

NES

PAX6

SOX1

⊖SS (Ex. 1)

-▲-FS (Ex. 3)

·■ LS (Ex. 2)

[FIG. 10]

Ectoderm

⊖SS (Ex. 1)

-▲-FS (Ex. 3)

·■ LS (Ex. 2)

Endoderm ▲ ⊖ Mesoderm

[FIG. 11]

Experimental Ex. 1    Experimental Ex. 2    Experimental Ex. 3    Experimental Ex. 4
Culture vessel    Culture vessel    Culture vessel    Culture vessel
(S1)    (L1)    (R1)    (V1)

[FIG. 12]

Example 5

Comparative Example 4

[FIG. 13]

[FIG. 14]

[FIG. 15]

[FIG. 16]

## INTERNATIONAL SEARCH REPORT

| International application No. |
|---|
| **PCT/JP2023/033295** |

| A. | CLASSIFICATION OF SUBJECT MATTER |
|---|---|

*C12N 5/10*(2006.01)i; *C07K 5/083*(2006.01)i; *C07K 5/087*(2006.01)i; *C07K 5/09*(2006.01)i; *C07K 5/097*(2006.01)i; *C07K 7/06*(2006.01)i; *C07K 7/64*(2006.01)i; *C12M 1/00*(2006.01)i; *C12N 1/00*(2006.01)i; *C12N 15/12*(2006.01)i; *C12N 15/86*(2006.01)i

FI: C12N5/10 ZNA; C12N15/86 Z; C12M1/00 A; C07K7/64; C07K5/087; C07K5/09; C07K5/083; C07K5/097; C07K7/06; C12N1/00 A; C12N1/00 F; C12N15/12

According to International Patent Classification (IPC) or to both national classification and IPC

| B. | FIELDS SEARCHED |
|---|---|

Minimum documentation searched (classification system followed by classification symbols)

C12N5/10; C07K5/083; C07K5/087; C07K5/09; C07K5/097; C07K7/06; C07K7/64; C12M1/00; C12N1/00; C12N15/12; C12N15/86

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | SUNG, T. -C. et al. Effect of cell culture biomaterials for completely xeno-free generation of human induced pluripotent stem cells. Biomaterials. 2020, vol. 230, no. 119638, pp. 1-13, supplementary pp. 1-5 | 1-4 |
|   | abstract, p. 2, column 2.2, p. 3, fig. 1, p. 4 to p. 5, column 2.8, supplementary, fig. 1, 2 |   |
| Y |   | 1-5 |
| Y | 熊谷 慎也, 81. 大気圧プラズマを用いた革新的遺伝子導入法の開発, 上原記念生命科学財団研究報告書, 2021, vol. 35, pp. 1-5, (KUMAGAI, Shinya), non-official translation (81. Development of innovative gene introduction method using atmospheric pressure plasma. Research Report of The Uehara Memorial Foundation.) p. 4 | 5 |
| Y | WO 2020/230885 A1 (SEKISUI CHEMICAL CO., LTD.) 19 November 2020 (2020-11-19) examples | 1-5 |

☐ Further documents are listed in the continuation of Box C.  ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **14 November 2023** | **28 November 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** |   |
|   | Telephone No. |

Form PCT/ISA/210 (second sheet) (January 2015)

## INTERNATIONAL SEARCH REPORT

| International application No. |
| --- |
| **PCT/JP2023/033295** |

**Box No. I        Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a. ☑ forming part of the international application as filed:

       ☑ in the form of an Annex C/ST.25 text file.

       ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

       ☐ in the form of an Annex C/ST.25 text file (Rule 13*ter*.1(a)).

       ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "In the form of an Annex C/ST.25 text file" above should be understood as "in ST.26 format".

Form PCT/ISA/210 (continuation of first sheet) (January 2015)

**INTERNATIONAL SEARCH REPORT**
Information on patent family members

International application No.

**PCT/JP2023/033295**

| Patent document cited in search report | | | Publication date (day/month/year) | Patent family member(s) | | | Publication date (day/month/year) |
|---|---|---|---|---|---|---|---|
| WO | 2020/230885 | A1 | 19 November 2020 | US | 2022/0325221 | A1 | |
| | | | | examples | | | |
| | | | | EP | 3971201 | A1 | |
| | | | | CN | 113166580 | A | |
| | | | | KR | 10-2022-0009367 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019162054 A
- WO 2020230885 A1

- WO 2018155595 A1

**Non-patent literature cited in the description**

- *The Journal of Cell Biology*, September 1995, vol. 130 (5), 1189-1196
- *Protein nucleic acid enzyme*, 2000, vol. 45 (15), 2477
- *Pathophysiology*, 1990, vol. 9 (7), 527-535

- *Journal of Osaka Women's and Children's Hospital*, 1992, vol. 8 (1), 58-66
- **ELAYNE M CHAN1 et al.** *Live cell imaging distinguishes bona fide human iPS cells from partially reprogrammed cells, nature biotechnology*, November 2009, vol. 27 (11)